# FASCICULE DE BREVET EUROPEEN

(11) **EP 1 709 027 B1**
(45) Date de publication et mention de la délivrance du brevet: **07.10.2009**
(21) Numéro de dépôt: 05717518.4
(22) Date de dépôt: 31.01.2005
(51) Int. Cl.: C07D 333/70, C07D 307/54, C07D 333/38, C07D 307/68, C07D 209/18, C07D 279/16, C07D 215/50, C07D 209/80, C07D 405/12, A61K 31/47, A61K 31/40, A61K 31/54, A61K 31/335, A61K 31/38, C07C 251/00

(54) **COMPOSES DE TYPE HYDRAZIDE ET LEUR UTILISATION DANS DES COMPOSITIONS PHARMACEUTIQUES POUR LE TRAITEMENT DES MALADIES CARDIOVASCULAIRES**
VERBINDUNGEN VOM HYDRAZIDTYP UND DEREN VERWENDUNG IN PHARMAZEUTISCHEN ZUSAMMENSETZUNGEN ZUR BEHANDLUNG VON HERZKREISLAUFERKRANKUNGEN
HYDRAZIDE TYPE COMPOUNDS AND THE USE THEREOF IN PHARMACEUTICAL COMPOSITIONS FOR THE TREATMENT OF CARDIOVASCULAR DISEASES

(30) Priorité: 30.01.2004 FR 0400913
(43) Date de publication de la demande: 11.10.2006
(60) Demande divisionnaire: 09166657.8
(73) Titulaire: Clinigenetics, 30000 Nîmes (FR)
(72) Inventeur: MARGUERIE, Gérard, F-94400 Vitry-sur-Seine (FR); MALAUD, Eric Résidence Le Marc-Aurèle, F-30900 Nîmes (FR)
(74) Mandataire: Agasse, Stéphane
(86) Numéro de dépôt international: PCT/FR2005/000199
(87) Numéro de publication internationale: WO 2005/082882

(56) Documents cités:
- US-A- 3 829 492
- PATENT ABSTRACTS OF JAPAN vol. 1999, no. 09, 30 juillet 1999 (1999-07-30) & JP 11 106371 A (NISSHIN FLOUR MILLING CO LTD), 20 avril 1999 (1999-04-20)
- TRIVEDI ET AL.: "Synthesis and antimicrobial activity of some heterocyclic compounds" IND. J. CHEM., vol. 32B, 1993, pages 497-500, XP001181798
- GEORGIEVA ET AL.: "Isonicotinoylhydrazone Analogs of Isoniazid: Relationship between Superoxide Scavenging and Tuberculostatic Acivities" BIOCHEMISTRY (MOSCOW), vol. 67, no. 5, 2002, pages 588-591, XP001181795
- PATEL ET AL.: "Studies on Anitubercular and Antibacterial Agents: prpearation of 1-(4-Amino benzoyl)-2-benzalhydrazine and 1-[4-(phenyl-thioureido)bezoyl]-2-substitu ted benzalhydrazine" J. IND. CHEM. SOC., vol. 61, 1984, pages 718-720, XP001181932
- SHAH ET AL.: "Sudies on isoniazide derivatives. Preparation and Antimicrobial Activity of 2-Aryl-3-(pyridylcarbamoyl)-5-carboxymethy l-4-thiazolidinones" J. IND. CHEM. SOC., vol. 62, no. 3, 1985, pages 255-257, XP001182001
- DAVE ET AL.: "Studies on Thiazolidinones as potential Antitubercular Compounds" J. INDIAN CHEM. SOC., vol. 63, no. 3, 1986, pages 320-322, XP001181800
- COWPER ET AL.: "Studies on Aminonitriles as Drug potentials : Aminonitries of Isoniazide" J. INST. CHEMISTS (INDIA), vol. 53, no. 7, 1981, pages 195-197, XP001181937
- GRAMMATICAKIS: "No 160. - Contribution a l'étude de l'absorption dans l?ultraviolet moyenet le visible des aryl- et aroyl-hydrazones. VII. - Nitrobenzoylhydrazones (o, m et p), 2e mémoire" BULLETIN DE LA SOCIETE CHIMIQUE DE FRANCE, no. 3, 1970, pages 933-945, XP001181938
- CHARY ET AL.: "Synthesis of 2-methyl-N-(4-oxo-2-aryl-3-thiazolidinyl)- 1,8-naphthyridine-3-carboxamides" SULFUR LETTERS, vol. 8, no. 2, 1988, pages 79-88, XP001181799
- SHILIKADZE ET AL.: BULLETIN OF THE ACADEMY OF SCIENCES OF THE GEORGIAN SSR, vol. 91, no. 1, 1978, pages 145-148, XP001182018
- SALEM: "Clinico pathological studies on some newly synthetic quinoline derivatives" J. DRUG RES. EGYPT, vol. 12, no. 1-2, 1980, pages 107-114, XP002284235
- CARIATI ET AL.: "Synthesis, structure, and Second-Order Nonlinear optical Properties of Copper(II) and Palladium(II) Acentric Complexes with N-Salicylidene-N'-aroylhydrazine Tridentate Ligands" INORGANIC CHEMISTRY, vol. 41, no. 25, 2002, pages 6597-6603, XP002284236
- KAUPPI ET AL.: "Targeting Bacterial Virulence: Inhibitors of Type III Secretion in Yersinia" CHEMISTRY & BIOLOGY, vol. 10, no. 3, mars 2003 (2003-03), pages 241-249, XP002284237

## Description

La présente invention concerne de nouveaux composés de type hydrazine qui sont utilisés comme agents actifs dans des compositions pharmaceutiques destinées notamment au traitement ou à la prévention des maladies cardiovasculaires.

Malgré une recherche pharmacologique très active et de grandes avancées dans les domaines chirurgicaux, les maladies cardiovasculaires, accidents coronariens et ischémies cérébrales, demeurent la cause principale des décès et des invalidités dans le monde industrialisé. Le diabète de type II et le syndrome métabolique qui lui est associé, l'hypercholestérolémie, l'obésité définie comme une augmentation de la masse graisseuse, l'hypercholestérolémie, l'hypertriglycéridémie, et la dyslipidémie athérogène caractérisée par des profils complexes de lipoprotéines sont les facteurs de risques reconnus de ces maladies cardiovasculaires.

Ces pathologies ont en commun un désordre du métabolisme des lipoprotéines. La dyslipidémie athérogène du diabétique de type II et du syndrome métabolique, par exemple, est caractérisée par un taux élevé de triglycérides (supérieur à 150 mg/dl), un taux de lipoprotéine de haute densité cholestérol bas (HDLc inférieur à 40 mg/dl), un taux de lipoprotéine de basse densité (LDLc) variable (inférieur ou supérieur à 100 mg/dl). Dans l'hypercholestérolémie, le taux circulant du LDL cholestérol est supérieur à 130 mg/dl, le taux de triglycéride est peu modifié voire normal. L'hypertriglycéridémie très souvent associée à l'obésité est caractérisée par une très forte augmentation des triglycérides (supérieur à 200 mg/dl) qui entre dans la structure des lipoprotéines.

La complication la plus sérieuse de tous ces syndromes est l'athérothrombose. L'athérothrombose est une pathologie complexe liée à ces désordres métaboliques et dont le développement est silencieux, progressif et peut débuter très tôt dans la vie en impliquant plusieurs phases successives.

La formation d'une plaque artérielle riche en lipide est un processus lent se développant en général sur plusieurs décennies. Il s'agit d'une accumulation progressive de lipoprotéines, de macrophages spumeux, et de calcium au niveau de la paroi artérielle. Les plaques affectent la plupart des individus soumis au régime riche en graisses animales habituel des pays occidentaux industrialisés, mais il existe une forte variabilité interindividuelle de la vitesse d'évolution et de l'extension des plaques qui est due en partie à des caractéristiques génétiques.

La présence de nombreux macrophages spumeux dans la plaque la rend vulnérable et occasionne des épisodes de rupture. La rupture de la plaque d'athérosclérose et la formation d'un thrombus plaquettaire sont quant à eux des processus aigus responsables des complications sévères de la maladie : l'infarctus coronaire et cérébral et la mort subite. La sévérité de la maladie dépend donc en grande partie de la taille de la plaque, de sa stabilité et de la manière dont le thrombus est formé par la rupture de cette plaque. Ce phénomène est assez mal connu et implique souvent un état inflammatoire chronique et une réponse immunologique. A ce jour, différentes options thérapeutiques sont disponibles pour le traitement de ces maladies.

Les agents hypolipémiants comme les statines ou l'ezetimibe, ont une efficacité reconnue. Les statines sont des inhibiteurs de la 3-hydroxy-methylglutaryl coenzyme A réductase qui est directement impliquée dans la synthèse du cholestérol. Les statines réduisent efficacement le taux de cholestérol et plus modestement le taux de triglycérides. L'ezetimibe inhibe l'absorption intestinale du cholestérol. Ces molécules sont donc préconisées en prévention primaire et secondaire pour la plupart des patients dont le taux de LDLc est élevé. Les essais cliniques ont cependant démontré que le bénéfice médical des agents hypolipémiants, concernant le risque cardiovasculaire, n'est que de 30 à 35%. Leur utilisation s'accompagne quelquefois d'effets secondaires non désirés qui imposent l'arrêt du traitement. Dans de nombreux cas, des atteintes musculaires, une toxicité hépatique et des phénomènes d'intolérance sont observés.

Les fibrates ou dérivés de l'acide fibrique sont également préconisés pour le traitement des dyslipidémies athérogène. Les dyslipidémies recouvrent des patients différents aux profils lipidique complexes : faible taux de cholestérol, taux élevés de triglycérides, faibles taux de HDLc. L'utilisation des fibrates réduit le risque d'accidents cardiovasculaires de 40 % environ. Leur utilisation s'accompagne malheureusement chez de nombreux patients d'effets non désirés dus à l'intolérance, une toxicité hépatique, et des atteintes musculaires.

L'accident thrombotique qui est la conséquence de la rupture d'une plaque artérielle, est en général traité par des agents antithrombotiques comme l'acide acétylsalicylique les thienopyridines ou les thyanopyridines.

Le document JP 11/106371 décrit des dérivés acylhydrazone qui peuvent être utilisés notamment pour le traitement de complications diabétiques diverses.

Il existe donc un besoin pour de nouveaux composés capables de soigner les maladies cardiovasculaires et notamment pour traiter la croissance et la vulnérabilité d'une plaque artérielle.

La présente invention a précisément pour objet de nouveaux composés de type hydrazide utiles comme agent actif dans des compositions pharmaceutiques notamment pour le traitement ou à la prévention des maladies cardiovasculaires.

On décrit des composés répondant à la formule générale (I) suivante : 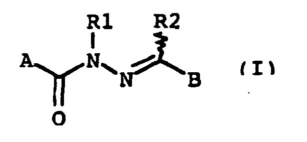 dans laquelle :
- R1 et R2, identiques ou différents, sont choisis parmi un atome d'hydrogène, un radical alkyle inférieur linéaire ou ramifié de 1 à 6 atomes de carbone, un radical fluoroalkyle de 1 à 6 atomes de carbones et de 3 à 7 atomes de fluor,
- A représente un groupement aromatique de un ou plusieurs cycles comprenant éventuellement un ou plusieurs hétéroatomes,
- B représente un groupement phényle éventuellement substitué ou un groupement pyridine éventuellement substitué.

Du fait de la fonction hydrazide au niveau de la double liaison N=C et de la signification des groupes B et R2, les composés de formule (I) peuvent se présenter sous des formes géométriques dites (E) ou (Z) existant soit en équilibre soit sous une forme unique préférentiellement (E) :
- forme (E) dans laquelle les groupements ACONR1 et B sont de part et d'autre de la fonction imine N=C, dite forme *trans,* ou
- forme (Z) dans laquelle les groupements ACONR1 et B sont d'un même côté de la fonction imine N=C, dite forme *cis*.
   Des composés préférés de formule (I) sont ceux dans lesquels B représente un groupement de formule (II) suivante : 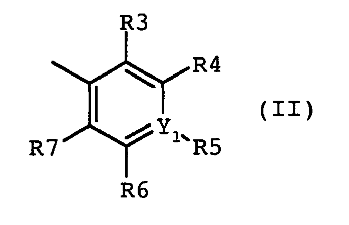
dans laquelle Y₁ est un atome de carbone pour former un noyau phényle ou un atome d'azote pour former un noyau pyridine et dans laquelle R3, R4, R5, R6 et R7, identiques ou différents, sont choisis parmi : un atome d'hydrogène, un atome d'halogène et plus particulièrement de fluor, chlore et brome, un groupe de formule -OH, -OR8 ou
- OCOR9, où R8 et R9 représentent un radical alkyl inférieur linéaire ou ramifié de 1 à 6 carbones, un groupe amino -NH₂ ou -N(r, r') dans lequel r et r' identiques ou différents représentent un radical alkyle inférieur linéaire ou ramifié, un radical aryle, ou un hétérocycle dans lequel r et r' pris ensemble forment un hétérocycle de taille variable, de préférence en position *para.*

Des composés préférés sont ceux de formule (I) dans laquelle R3 est un groupe de formule -OR8 et au moins deux des substituants R4, R5, R6 et R7 représentent un atome d'hydrogène. Parmi ceux-ci, on préfère également tout particulièrement les composés de formule (I) dans laquelle Y₁ est un atome de carbone.

D'autres composés préférés sont ceux de formule (I) dans lesquels B est de formule (II) dans laquelle Y₁ est un atome de carbone, répondant à la formule (I') suivante :

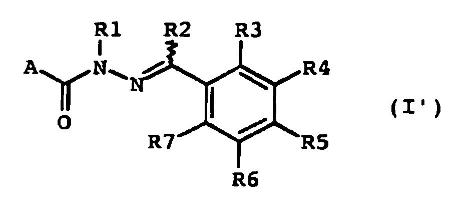

L'invention concerne des composés de formule (I) dans laquelle A représente un groupement de formule (III) suivante : 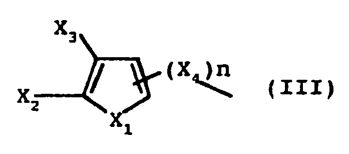 dans laquelle :
- X₁ est choisi parmi :
   - un atome d'oxygène et dans ce cas le groupement de formule (III) est un noyau 2-furanyl ou 3-furanyl en fonction de la position de la chaîne - (X₄)ₙ-acyl-hydrazide sur les carbones a ou β de cet hétérocycle,
   - un atome de soufre et dans ce cas le groupement de formule (III) est un noyau 2-thiophène ou 3-thiophène en fonction de la position de la chaîne -(X₄)ₙ-acyl-hydrazide sur les carbones α ou β, cet atome de soufre pouvant porter un atome d'oxygène pour former un sulfoxyde ou deux atomes d'oxygène pour former une sulfone,
   - un atome d'azote et dans ce cas le groupement de formule (III) est un noyau 2-pyrrol ou 3-pyrrol en fonction de la position de la chaîne acyl-hydrazide sur les carbones α ou β de cet hétérocycle, cet atome d'azote pouvant porter un atome d'hydrogène, un radical alkyle inférieur de 1 à 6 atomes de carbones, un radical fluoroalykyle ayant de 1 à 6 atomes de carbone et de 3 à 7 atomes de fluor, un radical acyle -COR10 dans lequel R10 représente une chaîne alkyle linéaire ou ramifiée de 1 à 6 carbones, ou un radical aryle ou aralkyle,
- X₂ et X₃, identiques ou différents, sont choisis parmi :
   - un atome d'hydrogène, une chaîne alkyle linéaire ou ramifiée inférieure de 1 à 6 atomes de carbone, un radical fluoroalkyle ayant de 1 à 6 atomes de carbone et de 3 à 7 atomes de fluor,
   - un atome d'halogène, préférentiellement un atome de fluor, de chlore ou de brome,
   - un groupe nitro -NO₂, un groupe amino -NH₂ ou un groupe -N(r, r'), dans lequel r et r' identiques ou différents représentent un radical alkyle inférieur linéaire ou ramifié, un radical aryle, ou un hétérocycle dans lequel r et r' pris ensemble forment un hétérocycle de taille variable,
   ou encore X₂ et X₃ sont inclus dans un cycle aromatique de type benzénique ou aza-benzénique si ce cycle comporte un atome d'azote, pour former un hétérocycle aromatique de type benzofurane lorsque X₁ est un atome d'oxygène, un noyau benzopyrrole lorsque X₁ est un atome d'azote libre ou substitué comme ci-dessus, un noyau benzothiophène lorsque X₁ est un atome de soufre libre ou substitué comme ci-dessus ou encore un noyau de type pyridino si un atome d'azote intracyclique est présent,
- n est 0 ou 1,
- X₄, s'il est présent, représente un groupe -CH₂-, OCH₂-, ou -CH=CH-.

Des composés de formule (I) dans lesquels B est un groupement de formule (II) et A un groupement de formule (III) répondent à la formule (IV) suivante : 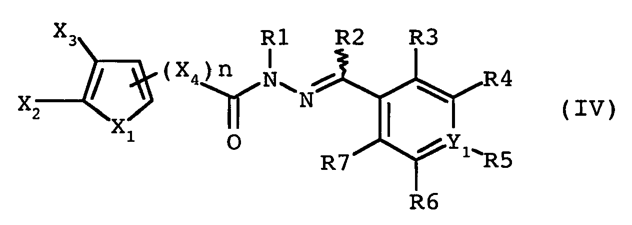 dans laquelle Y₁, X₁, X₂, X₃, R1 et R2 ont la même signification que précédemment et R3 à R7 identiques ou différents, sont choisis parmi : un atome d'hydrogène, un atome d'halogène et plus particulièrement de fluor, chlore et brome, un groupe de formule -OH, -OR8 ou
- OCOR9, où R8 et R9 représentent un radical alkyl inférieur linéaire ou ramifié de 1 à 6 carbones, un groupe amino -NH₂ ou -N(r, r') dans lequel r et r' identiques ou différents représentent un radical alkyle inférieur linéaire ou ramifié, un radical aryle, ou un hétérocycle dans lequel r et r' pris ensemble forment un hétérocycle de taille variable, de préférence en position para,
- n est 0 ou 1,
- X₄, s'il est présent, représente un groupe -CH₂-, - OCH₂-, ou -CH=CH-.

Des composés préférés sont ceux de formule (IV) dans laquelle R3 est un groupe de formule -OR8 et au moins deux des substituants R4, R5, R6 et R7 représentent un atome d'hydrogène. Parmi ceux-ci, on préfère également tout particulièrement les composés de formule (IV) dans laquelle Y₁ est un atome de carbone.

Parmi les composés de formules (IV), l'invention se rapporte plus particulièrement aux composés suivants :
- N'-[(*1E*)-(2-hydroxy-4,6-diméthoxyphényl)méthylène]-1-benzothiophène-2-carbohydrazide (désigné CGP02-01),
- (*2Z*)-3-(2-furyl)-N'-[(*1E*)-(2-hydroxy-4,6-diméthoxyphényl)méthylène] acrylohydrazide (désigné CGP02-02),
- N'-[(1E)-(2-hydroxy-4,6-diméthoxyphényl)méthylène]-5-méthylthiophène-2-carbohydrazide (désigné CGP02-03),
- 2-furancarboxylique acide (2-hydroxy-4,6-diméthoxy-benzylidène)-hydrazide (désigné CGP02-07),
- (1H-indol-3-yl) acétique acide(2-hydroxy-4,6-diméthoxybenzylidène)-hydrazide (désigné CGP02-08),
- benzo[b]thiophène-2-carboxylique acide (3,5-dibromo-2-hydroxy-benzylidene)-hydrazide (désigné CGP02-18).

Parmi ceux-ci, on préfère tout particulièrement le N'-[(*1E*)-(2-hydroxy-4,6-diméthoxyphényl)méthylène]-1-benzothiophène-2-carbohydrazide (CGP02-01).

Une autre forme de réalisation de l'invention concerne des composés de formule (I) dans laquelle A représente un groupement de formule (IX) suivante : 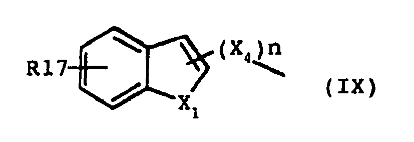 dans laquelle :
- X₁ et X₄ ont la même signification que précédemment,
- n est 0 ou 1,
- R17 est choisi parmi :
   - un atome d'hydrogène, un radical alkyle inférieur linéaire ou ramifié de 1 à 6 atomes de carbone, un radical fluoroalkyle de 1 à 6 atomes de carbones et de 3 à 7 atomes de fluor,
   - un atome d'halogène, préférentiellement un atome de fluor, de chlore ou de brome,
   - un groupement OR' pour lequel R' inférieur linéaire ou ramifié de 1 à 6 atomes de carbone, un radical fluoroalkyle de 1 à 6 atomes de carbone et de 3 à 7 atomes de fluor.

Des composés de formule (I) dans lesquels B est un groupement de formule (II) et A est un groupement de formule (IX) répondent à la formule (X) suivante : 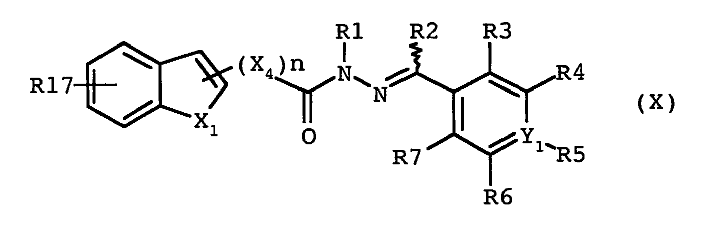 dans laquelle Y₁, R1, R2, R17, X₁, X₄ et n ont la même signification que précédemment et R3, R4, R5, R6 et R7 identiques ou différents, sont choisis parmi : un atome d'hydrogène, un atome d'halogène et plus particulièrement de fluor, chlore et brome, un groupe de formule -OH, -OR8 ou -OCOR9, où R8 et R9 représentent un radical alkyl inférieur linéaire ou ramifié de 1 à 6 carbones, un groupe amino -NH₂ ou -N(r, r') dans lequel r et r' identiques ou différents représentent un radical alkyle inférieur linéaire ou ramifié, un radical aryle, ou un hétérocycle dans lequel r et r' pris ensemble forment un hétérocycle de taille variable, de préférence en position para.

Des composés préférés sont ceux de formule (X) dans laquelle R3 est un groupe de formule -OR8 et au moins deux des substituants R4, R5, R6 et R7 représentent un atome d'hydrogène. Parmi ceux-ci, on préfère également tout particulièrement les composés de formule (X) dans laquelle Y₁ est un atome de carbone.

L'invention concerne aussi, quand cela est possible, les sels des composés ci-dessus avec des acides de type pharmaceutique tolérés physiologiquement.

A titre d'exemple de sels pharmaceutiques physiologiquement acceptables, on peut citer de manière non limitative les sels d'acide acétique, chlorhydrique, cinnamique, citrique, formique, hydrobromique, hydroiodique, hydrofluorique, malonique, méthanesulfonique, oxalique, picrique, maléique, lactique, nicotinique, plénylacétique, phosphorique, succinique, tartrique, les sels d'ammonium, de diéthylamine, de pipérazine, de nicotinamide, d'urée, de sodium, de potassium, de calcium, de magnésium, de zinc, de lithium, de méthylamino, de diméthylamino, de triméthylamino, de tris(hydroxyméthyl)aminométhane.

L'invention concerne les compositions pharmaceutiques pour l'homme ou l'animal comprenant à titre d'agent actif au moins un des composés décrits ci-dessus ou leur sel pharmaceutiquement acceptable.

En effet, ces composés sont utiles pour le traitement de l'athérosclérose et de la resténose artérielle. Ils ont la propriété de diminuer la prise de poids due à une accumulation de graisse abdominale, de diminuer l'augmentation du taux de cholestérol total et du cholestérol libre et le dépôt de triglycérides au niveau de la paroi artérielle et de réduire l'accumulation de macrophages au niveau des plaques athéromateuses. Ces composés ont en particulier la propriété d'inhiber la formation de cellules macrophages spumeuses en inhibant l'accumulation de vésicules lipidiques intra-cellulaire. Par extension, ces molécules sont donc capables de traiter l'obésité, le diabète de type II, l'ischémie cérébrale et les stéatoses hépatiques, en bloquant l'accumulation de vésicules lipidiques dans des cellules telles que l'hépatocyte, la cellule du muscle lisse, l'adipocyte et la cellule endothéliale.

Ces composés sont donc utiles comme agents actifs dans des méthodes ou des compositions pharmaceutiques pour le traitement et éventuellement la prévention de toutes les maladies associées à des désordres du métabolisme des lipides. A ce titre, on peut citer entre autres, l'hypercholestérolémie, l'hypertriglycéridémie , la dyslipoproteinémie, la chylomicronémie, la lipodistrophie, l'hyperglycémie, ainsi que les pathologies associées à ces dysfonctionnements : l'athérosclérose, l'obésité, le diabète de type II, et la résistance à l'insuline, l'insuffisance cardiaque et l'ischémie cérébrale (stroke).

Par ailleurs, ces composés ayant la propriété de réduire le rétrécissement de la paroi artérielle, ils sont utiles comme agents actifs dans des méthodes ou des compositions pharmaceutiques pour le traitement et éventuellement la prévention de la resténose.

Les compositions pharmaceutiques selon l'invention comprennent des quantités suffisantes d'au moins un composé décrit précédemment.

Sur la base des résultats obtenus *in vivo* et présentés dans la partie expérimentale ci-après, les compositions de l'invention peuvent être administrées dans le cadre d'un traitement en une plusieurs doses de 0,01 à 500 milligrammes par kilogramme de poids du corps par jour d'un ou plusieurs composés de l'invention.

La formulation des compositions pharmaceutiques selon l'invention est du type généralement utilisée dans le domaine pharmaceutique.

A titre d'exemple, il peut s'agir de vecteurs pharmaceutiques tels que, par exemple, des sels ou des électrolytes, des sels de l'acide scorbique, de l'eau ou des solutions tamponnées, des solutions colloïdales, des substances à base de cellulose, du polyéthylène glycol, des polyacrylates, des cires, des protéines, ou tout autre substance capable de dissoudre ou de rendre le composé actif disponible pour une action thérapeutique.

Les compositions de la présente invention peuvent être administrées sous forme injectable ou par voie orale, parentérale, nasale sous forme de spray, rectale ou vaginale, par l'implantation de réservoir ou dispensateurs ou sous tout autre forme galénique en usage dans le domaine pharmaceutique.

Les formes injectables de ces compositions peuvent être des suspensions aqueuses ou oléagineuses. Ces suspensions peuvent être formulées selon tout procédé en usage dans ce domaine en utilisant des solvants ou des diluants non toxiques comme par exemple le 1,3-butanediol. Parmi les solvants acceptables, il est possible d'utiliser de l'eau, des solutions tamponnées, des solutions de Ringer, ou des solutions isotoniques de sels. D'autres diluants acceptables peuvent être constitués de mono ou di-glycérides synthétiques, des alcools à longue chaîne, ou des dispersants tels que la carboxymethyl cellulose ou tout autre diluant ou émulsifiant utilisés dans la formulation de suspension pharmaceutique.

Les compositions pharmaceutiques de la présente invention administrées par voie orale, peuvent être sous forme de capsule, de cachet ou de suspensions aqueuse ou sous forme d'émulsion. Ces formulations peuvent éventuellement contenir des composés chimiques destinés à adoucir ou améliorer le goût.

Les compositions pharmaceutiques de la présente invention peuvent être administrées sous forme de suppositoire en mélangeant le produit avec un excipient non irritant, non allergique, solide à la température ambiante et liquide à la température rectale de manière à libérer le composé actif. De telles formulations peuvent utiliser par exemple de la cire d'abeille, des polyéthylèneglycols ou du beurre de cacao.

Ces compositions pharmaceutiques peuvent également comprendre une combinaison d'un plusieurs composés de l'invention avec une ou plusieurs autres molécules thérapeutiques. Ces molécules peuvent être par exemple des agents hypolipémiant réduisant la synthèse du cholestérol comme les « statines », des inhibiteurs de l'enzyme de conversion de l'angiotensine II comme par exemple le Losartan, des anticalciques, des antithrombotiques, des bêta-bloquants, des inhibiteurs des membres de la famille des récepteurs activés des proliférateurs du péroxisome (famille des PPAR), des inhibiteurs de la synthèse ou du métabolisme des triglycérides comme les Fénofibrate, des agents capables d'augmenter la résistance à l'insuline comme les Troglitazone ou les Pioglitazone et d'une manière générale, toute autre molécule capable d'améliorer les performances pharmacologiques des composés décrits dans la présente invention.

L'invention concerne également l'utilisation d'un composé selon l'invention pour la préparation d'une composition pharmaceutique selon l'invention.

L'invention concerne également l'utilisation d'un composé selon l'invention pour la fabrication d'un médicament.

On décrit également la préparation des composés de formule (I) et des compositions pharmaceutiques contenant comme principe actif au moins un desdits composés.

Les composés de formule (I) peuvent être préparés selon les techniques connues de l'homme de métier. La présente invention décrit à cet égard une voie de synthèse générale qui est illustrée par le schéma ci-dessus et dans l'exemple de mode opératoire qui suit où les composés de départ sont obtenus dans le commerce ou peuvent être synthétisés selon des procédés habituels connus de l'homme de métier et décrits dans les livres classiques de chimie organique ("Advanced Organic Chemistry" de M. B. Smith & J. March, Ed. John Wiley & Sons, "Handbook of Heterocyclic Chemistry" de A. R. Katritzky, Ed. Pergamon, et "Heterocyclic Chemistry" de J. A. Joule et K. Mills, Ed. Blackwell Science).

Il est entendu que la description n'est pas limitée à une voie de synthèse particulière, et s'étend à d'autres procédés permettant la production des composés de formule (I). A titre d'exemple, les composés de formule (I) peuvent ainsi être préparés soit en phase liquide soit en phase parallèle sur support solide. Les méthodes ci-dessous sont données à titre non limitatif, et tous autres procédés permettant de créer des doubles liaisons de type imines N=C substituées peuvent êtres utilisés pour préparer les composés de l'invention.

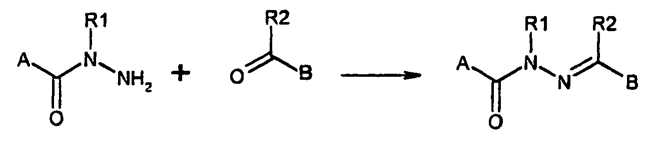

Dans le schéma ci-dessus, R1, R2, A et B ont la même signification que précédemment.

Selon le schéma ci-dessus, les composés de formule (I) sont directement préparés par une réaction de condensation entre d'une part la matière première désignée carbo-hydrazide représentée par la formule A-CO-NR1-NH2 et un aldéhyde ou une cétone représentée par la formule R2-COB, pour lesquels les groupes A et R1 d'une part et B et R2 d'autre par ont respectivement les significations décrites pour les formules (II) à (IV). Ces matières premières de départ mises en jeu sont commerciales et peuvent être acquises auprès de sociétés de chimie à façon telles que Maybridge (Grande-Bretagne) ou Pfaltz-Bauer (USA), ce choix de sociétés n'étant pas exclusif.

Cette réaction de condensation est conduite préférentiellement en atmosphère inerte, entre 0°C et 50°C de préférence à température ambiante en présence d'une base organique amine tertiaire de préférence la base de Hünig di-isopropyléthylamine DIEA, dans un solvant dipolaire aprotique de préférence le diméthylformamide DMF anhydre ou dans l'éthanol à reflux pendant 6 à 8 heures. Le monitoring de l'avancement de la réaction est réalisé par analyse HPLC permettant de contrôler le temps de réaction préférentiellement inférieur à 24 h.

D'autres avantages et caractéristiques de l'invention apparaîtront des exemples qui suivent et dans lesquels il sera fait référence aux dessins en annexe où :

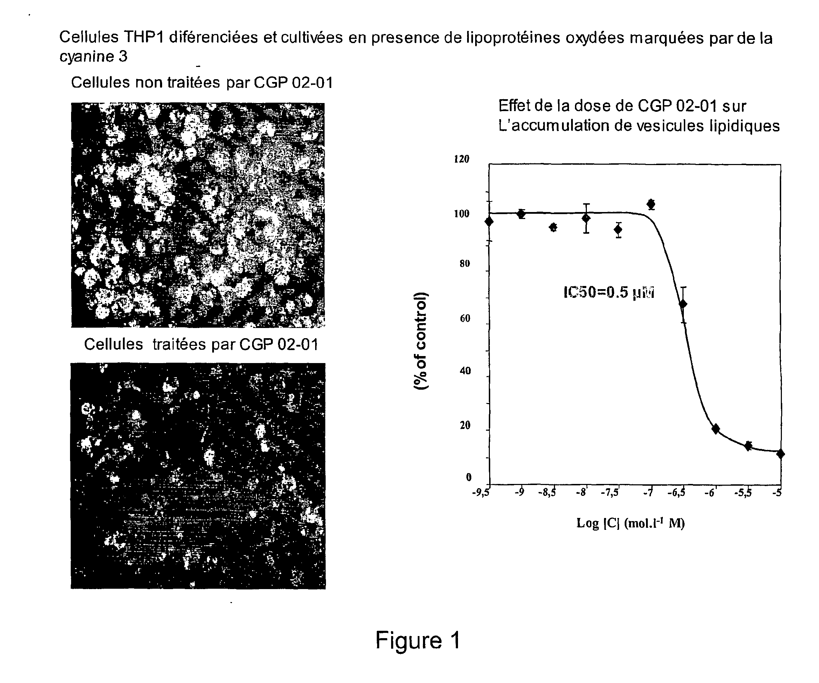

- La figure 1 représente les effets de doses croissantes du composé CGP02-01 sur l'accumulation de vésicules lipidiques dans une cellule macrophage cultivée en présence de lipoprotéines marquées à l'aide de l'agent fluorescent Cyanine 3. La courbe dose réponse indique une IC50 de 5 x 10⁻⁷ M.

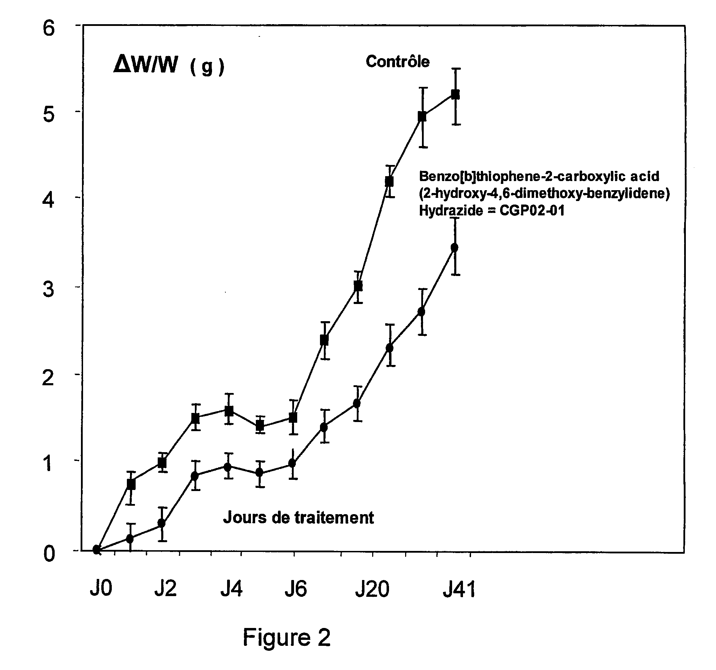

- La figure 2 montre la réduction de la prise de poids par réduction de la masse graisseuse abdominale chez des souris ApoE négative après traitement par le composé CGP02-01. Les souris ont été traitées pendant 41 jours à une dose de 20µg de composé CGP02-01 par jour. Les souris contrôles et les souris traitées ont été alimentées par un régime normal sans surcharge en cholestérol.

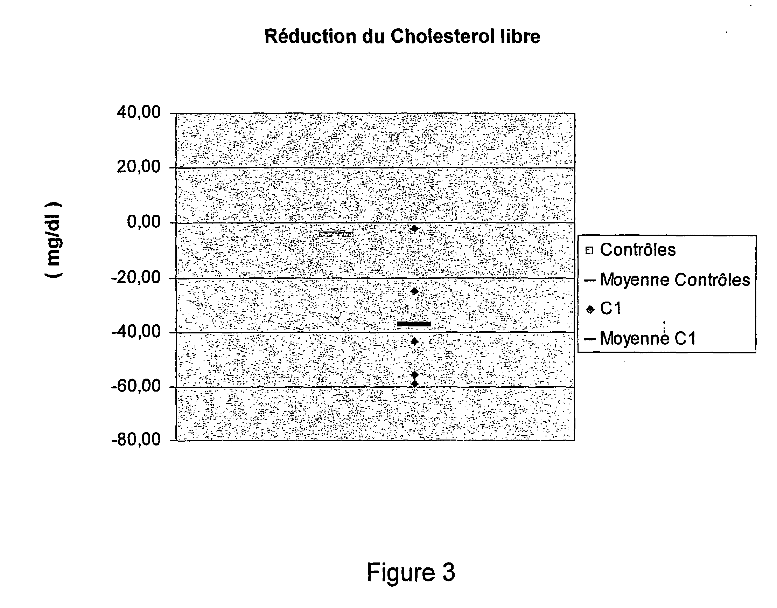

- La figure 3 représente l'effet du composé CGP02-01 sur l'augmentation du taux de cholestérol libre dans le plasma chez des souris ApoE négative. Les souris ont été traitées d'une manière identique à celle qui est décrite dans la figure 2.

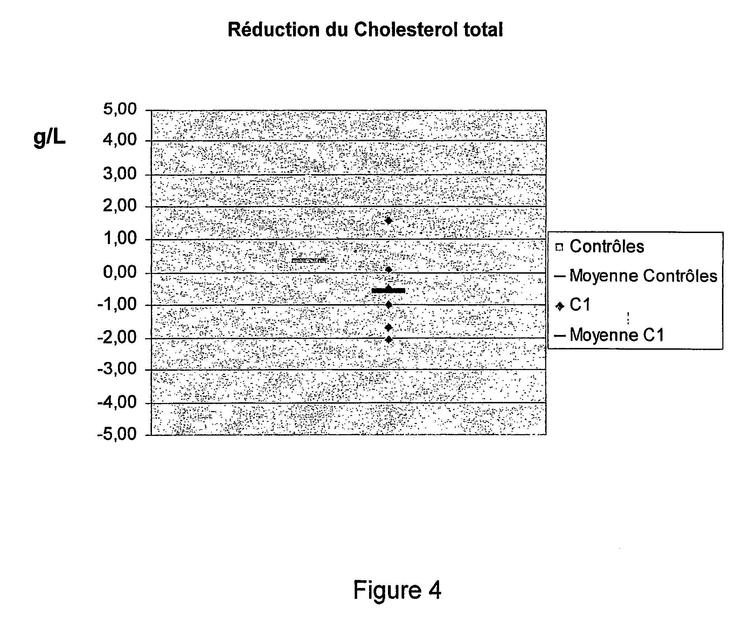

- La figure 4 montre la variation du taux de cholestérol total chez une souris ApoE négative traitée ou non traitée par le composé CGP02-01.

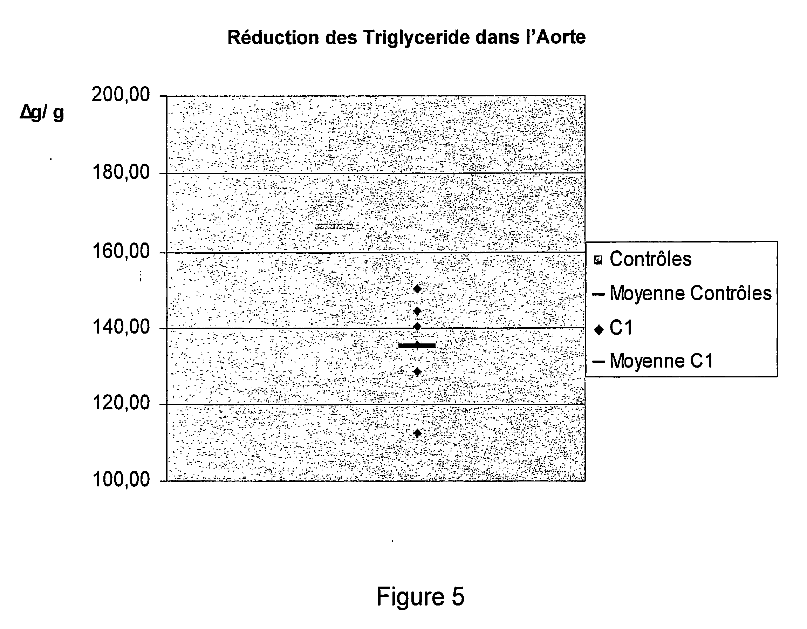

- La figure 5 montre la variation du taux de triglycérides présent dans l'aorte de souris ApoE négative traitées ou non traitées par le composé CGP02-01.

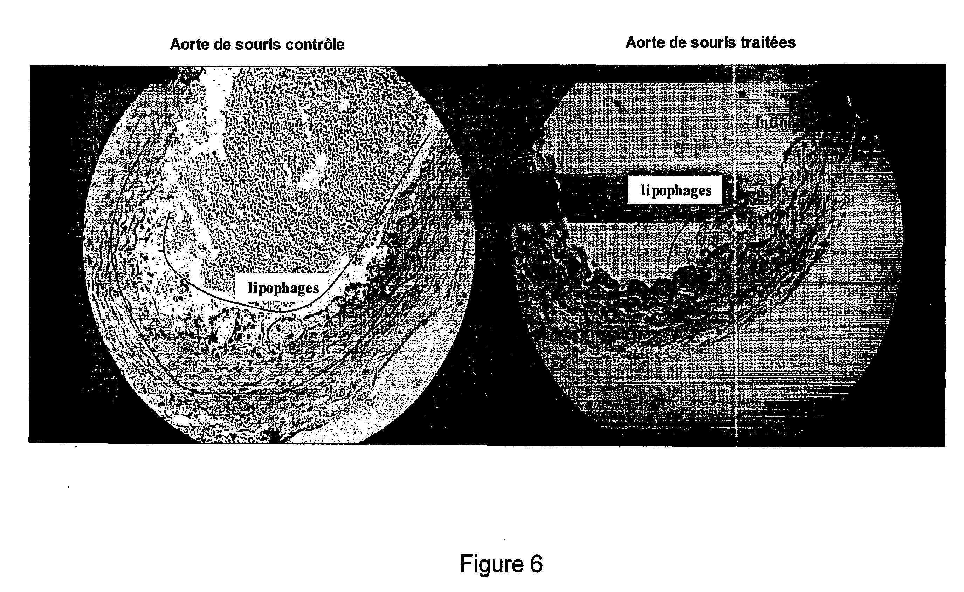

- La figure 6 montre la modification de la plaque d'athérome chez des souris ApoE négative traitées ou non traitées par le composé CGP02-01. On note la présence d'une situation inflammatoire et de nombreux macrophages spumeux dans la lésion des souris non traitées, et la réduction significative de ces macrophages ainsi que l'absence de réaction inflammatoire dans l'aorte des souris traitées.

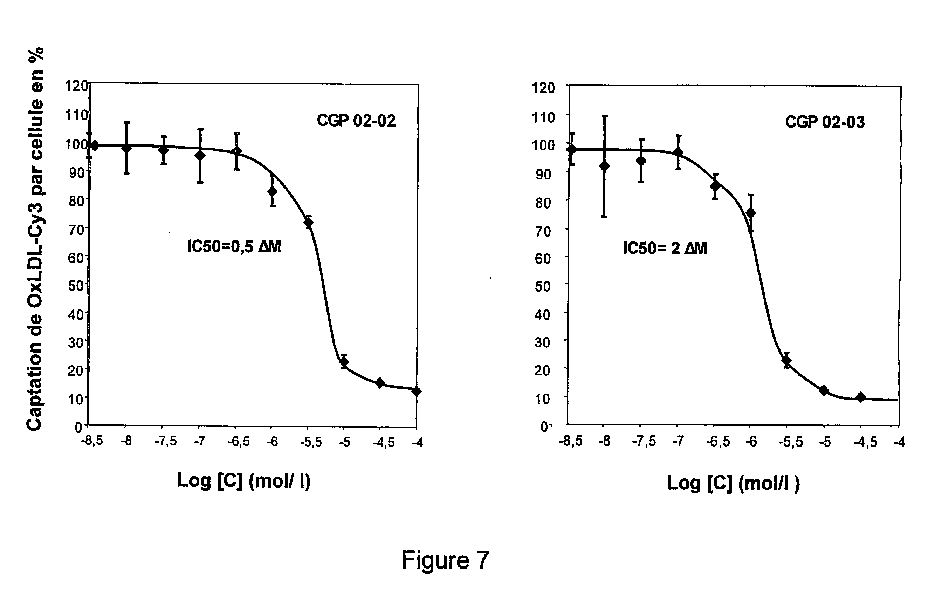

- La figure 7 montre l'inhibition de la formation de cellules spumeuses par les composés CGP 02-02 et CGP 02-03. Les cellules THP1 différenciées sont cultivées en présence de lipoprotéines oxydées (3 µg/ml oxLDL) marquées par de la cyanine 3 pendant 24 heures à 37°C. Les cellules sont traitées par les composés CGP 02-02 et CGP 02-03 à différentes concentrations. Les conditions sont identiques à celles de la figure 1.

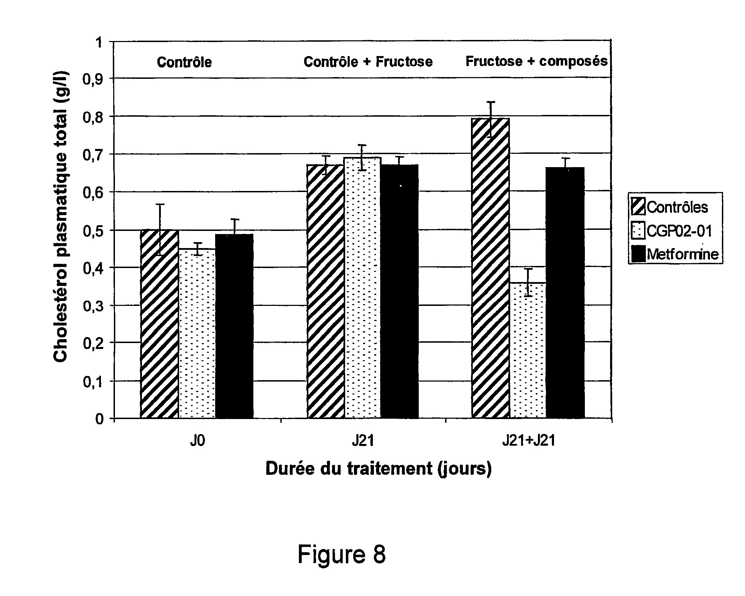

- La figure 8 représente l'effet du composé CGP 02-01 administré par voie orale (50 mg/kg) sur le taux plasmatique de cholestérol total (g/L) après 3 semaines de traitement d'un modèle de rat (n=12) ,soumis à un régime riche en fructose (10 %). La metformine a été injectée à la même dose pour servir de témoin standard dans ce modèle animal.

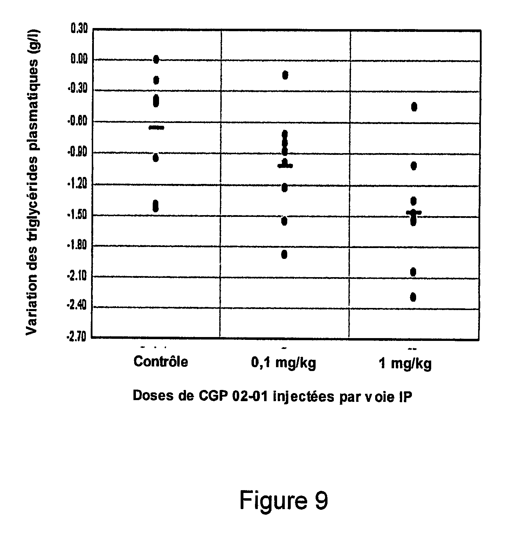

- La figure 9 illustre les variations du taux plasmatique de triglycérides (g/L) chez une souris ApoE -/soumise à un régime riche en cholestérol et traitée pendant 3 mois avec le composé CGP 02-01.

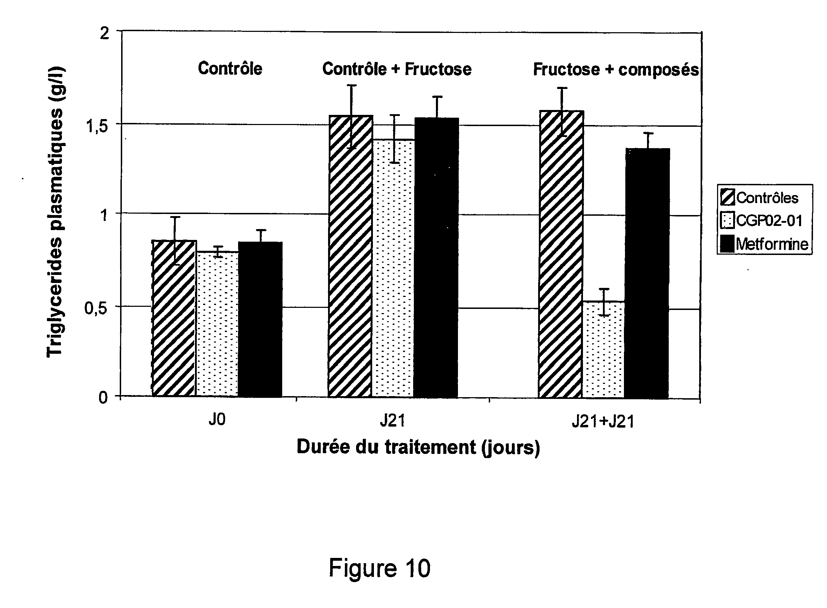

- La figure 10 montre l'effet du composé CGP 02-01 administré par voie orale (50 mg/kg) sur le taux plasmatique de triglycérides (g/L) après 3 semaines de traitement sur un modèle de rat (n=12), soumis à un régime riche en fructose (10 %). Les rats sont nourris quotidiennement avec un régime contenant 10% de fructose pendant 3 semaines. Le régime est ensuite maintenu en ajoutant le composé CGP 02-01 pendant 3 semaines. Chaque rat est analysé séparément. La metformine a été administrée à la même dose pour servir de témoin standard dans ce modèle animal

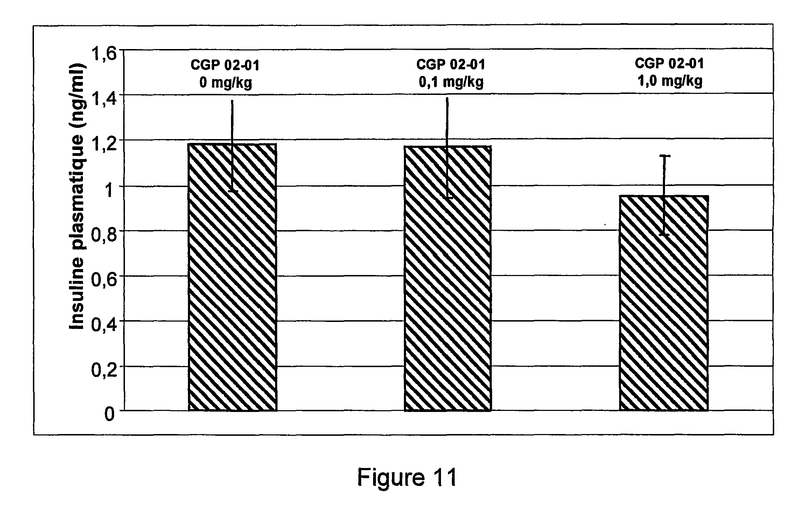

- La figure 11 représente l'effet du composé CGP 02-01 injecté par voie IP sur le taux plasmatique d'insuline (ng/mL) de souris ApoE -/- soumises à un régime riche en cholestérol. Les souris sont traitées pendant 3 mois.

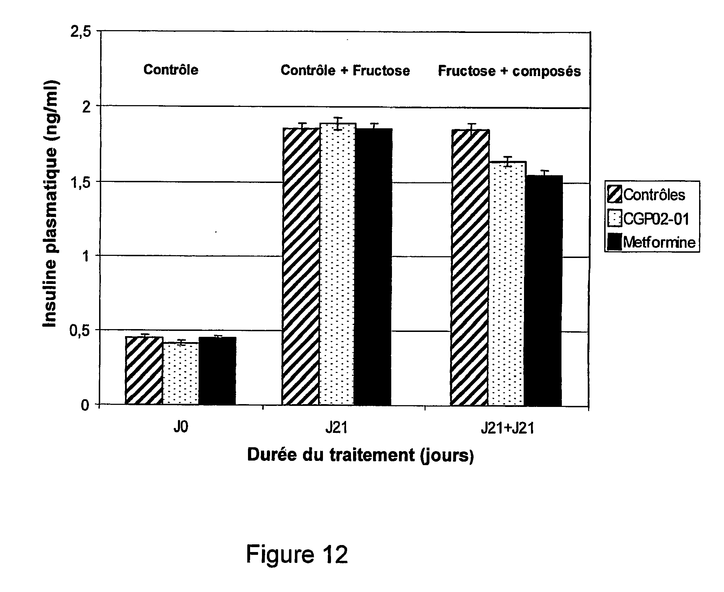

- La figure 12 montre l'effet du composé CGP 02-01 administré par voie orale (50 mg/kg) sur le taux plasmatique d'insuline (ng/mL) après 3 semaines de traitement sur un modèle de rat (n=12), soumis à un régime riche en fructose (10 %). Les rats sont nourris quotidiennement avec un régime contenant 10% de fructose pendant 3 semaines. Le régime est ensuite maintenu en ajoutant le composé CGP 02-01 pendant 3 semaines. Chaque rat est analysé séparément. La metformine a été administrée à la même dose pour servir de témoin standard dans ce modèle animal.

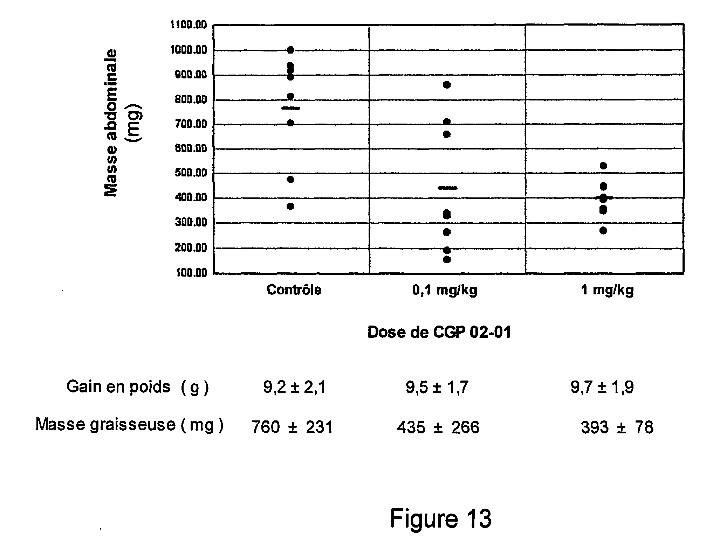

- La figure 13 montre l'effet du composé CGP 02-01 injecté par voie IP à différentes doses sur l'obésité abdominale de souris ApoE -/- soumises à un régime riche en cholestérol. Les souris sont traitées pendant 3 mois.

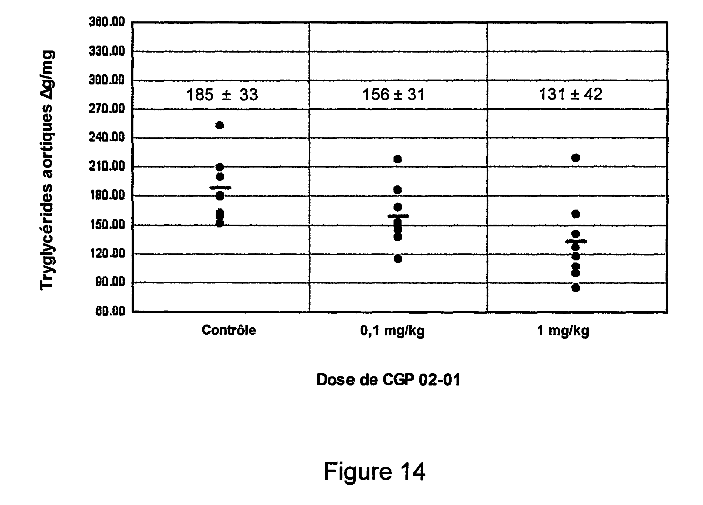

- La figure 14 montre l'effet du composé CGP 02-01 injecté par voie IP à différentes doses sur le dépôt de triglycérides dans la paroi aortique de souris ApoE -/soumises à un régime riche en cholestérol. Les souris sont traitées pendant 3 mois.

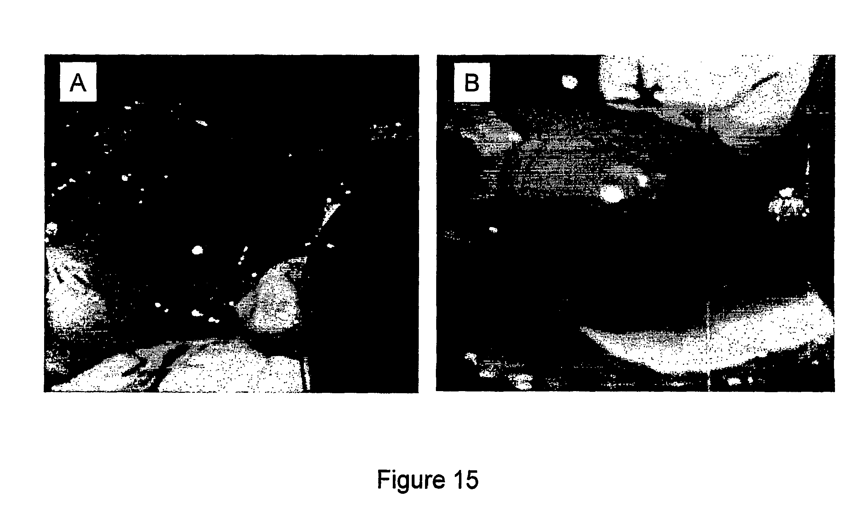

- La figure 15 montre que lorsque des souris ApoE -/sont soumises à un régime normal ou riche en cholestérol, elles développent une ischémie coronarienne illustrée par la présence d'une micro-embolisation des microvaisseaux coronariens (figure A). Lorsque le composé CGP 02-01 est injecté à ces souris, ces lésions cardiaques sont considérablement réduites (figure B).

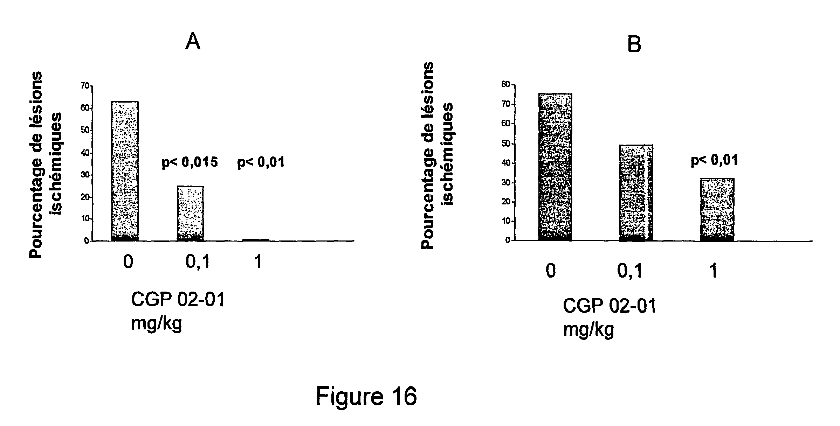

- La figure 16 illustre l'effet dose du composé CGP 02-01 sur les lésions coronariennes de souris ApoE -/soumises à un régime normal (figure A) et à un régime riche en cholestérol (figure B).

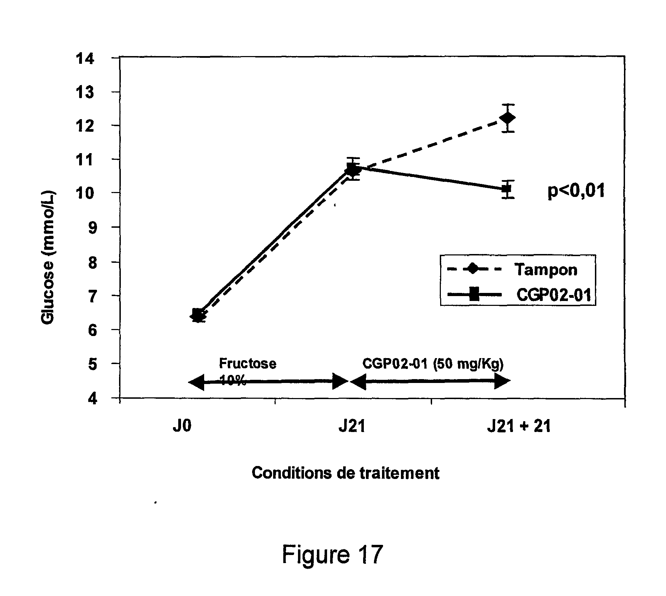

- La figure 17 illustre l'effet du composé CGP 02-01 sur l'augmentation de la glycémie chez des rats soumis à un régime riche en fructose (10%). Le régime est maintenu pendant 21 jours. Le composé est administré par voie orale suite à la période de diète fructose. La figure illustre l'effet stabilisant du composé.

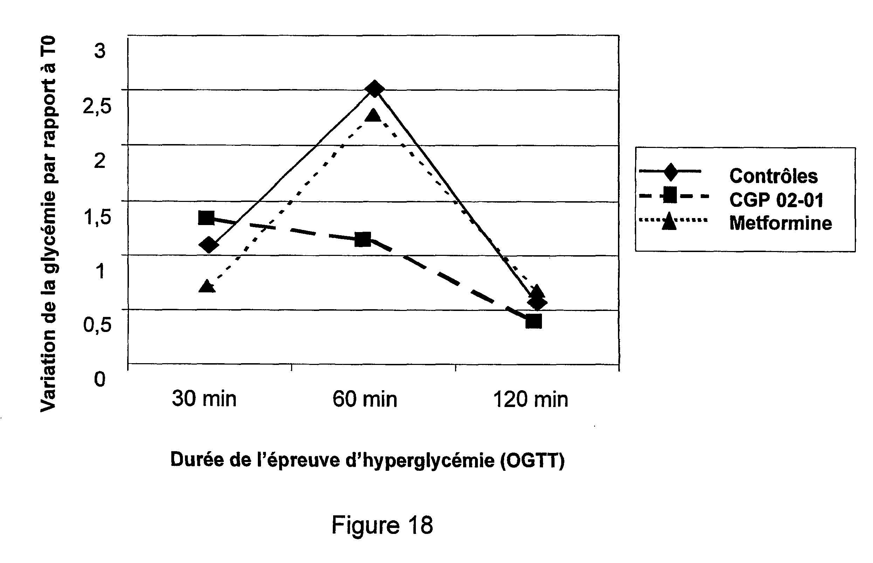

- La figure 18 illustre l'effet bénéfique du composé CGP 02-01 sur la tolérance au glucose chez des rats soumis à un régime riche en fructose (10%). Le composé est administré par voie orale, au bout du 14éme jour une dose unique supplémentaire de 2 g/kg de glucose est administrée. La glycémie est mesurée à 30, 60 et 120 minutes après ce choc glycémique.

### Exemple 1 Synthèse du CGP02-01.

Dans un tricol sec équipé d'une agitation magnétique, sont introduits 510,08 mg de l'acide [benzo(b)thiophène]-2-carboxylique hydrazide commercial dissout dans 28 ml de DMF anhydre. Après addition de 256 µl de DIEA (diéthylisopropylamine), la solution est agitée à température ambiante pendant 5 min. A cette solution légèrement colorée jaune sont additionnés 538,27 mg de 4,6-diméthosysalicyl-aldéhyde, le milieu est agité à température ambiante pendant 24h. L'avancement de la réaction est suivi par analyse HPLC jusqu'à consommation complète de la matière première. Après évaporation du solvant, le résidu solide obtenu est recristallisé dans CH₃CN puis lavé à l'éther éthylique. Le produit purifié obtenu N'-[(1E)-(2-hydroxy-4,6-diméthoxyphényl)méthylène]-1-benzothiophène-2-carbohydrazide est un solide jaune (743,6 mg, rdt = 71%).

### Données physico-chimiques :

Masse moléculaire : 356,40 g/mol
Point de fusion : 205,4°C
Pureté LC-MS : 100% (M+1 = 357,33)
Pureté HPLC : 95,8% (temps de rétention : 20 min, détection UV : 200-400 nm)
RMN 1H (DMSO-*d6*; 400MHz) : δ (ppm) 3,799 (s, 3H, OCH3), 3,862 (s, 3H, OCH3), 6,16 (s, 1H, Ar), 6,17 (s, 1H, Ar), 7,495 (m, 2H, Ar), 8,02 (dd,1H J=7,2 Hz et 1,3 Hz), 8,07 (dd, 1H, Ar, J=7,2 Hz et 1,4 Hz), 8,231 (s, 1H, CH=C), 8,861 (s, 1H, CH=N), 12,26 (s, 1H, OH), 12,348 (s, 1H, N-NH-CO).
RMN 13C (DMSO-*d6*, 400 MHz) : δ (ppm) 55,438 (OCH3), 55,948 (OCH3),90,524 (CH, Ar), 93,843 (CH, Ar), 122,846 (CH, Ar), 125,097 (CH, Ar), 124,439 (2CH, Ar), 125,684 (CH=C), 126,577 (CH=N), 145,980 (CO-NH=N).
IR-FT (KBr 0,05%): 3445,66 (Ar-OH), 1630,21 (-CO-NH=N), 1600,27 (-NH-N=C-) cm⁻¹.

### Analyse élémentaire : C₁₈H₁₆N₂O₄S+0, 5 H₂O

| | %C | % H | % N | % S |
|---|---|---|---|---|
| Théorique | 59,17 | 4,69 | 7,67 | 8,77 |
| Trouvée | 59,40 | 4,66 | 7,83 | 8,79 |

### Exemple 2 : Cultures cellulaires.

Plusieurs lignées de cellules permanentes peuvent être utilisées pour démontrer l'effet des molécules de la famille à laquelle appartient la molécule CGP02-01 sur la fixation et l'accumulation de lipides dans des vésicules intracellulaires. Ces cellules peuvent incorporer des lipoprotéines, des lipoprotéines modifiées, par exemple oxydées ou acétylées, des triglycérides ou des chylomicrons. Ces cellules ont la capacité de se transformer en cellules spumeuses et peuvent ainsi présenter un phénotype athérogène. Il est possible d'utiliser, à titre d'exemple, les cellules THP1, U937, KG1 ou tout autre cellule pouvant être activée et différentiée en macrophage, cellule endothéliale, cellule du muscle lisse, hépatocyte ou adipocyte puis mise en culture en présence de milieu contenant des lipoprotéines.

D'autres types de cellules ayant été génétiquement modifiées pour exprimer des récepteurs membranaires spécifiques de la captation de lipoprotéines ou des acides gras peuvent également être utilisées. Ces récepteurs membranaires peuvent faire partie de la famille des molécules scavenger contenant des protéines telles que SRAI, SRAII, SRBI, CD36 ou des membres de la famille des récepteurs des acides gras (FABP) par exemple.

A titre d'exemples, on peut citer plus particulièrement des cellules du type des cellules THP1 différenciées sous l'action de phorbol 12-myristate-13-acetate ( PMA ) à une concentration de 10⁻⁷ M, ont été utilisées pour mesurer la formation et l'accumulation de vésicules lipidiques observées au cours de la formation de macrophage spumeux en présence ou en absence du composé CGP02-01.

Les cellules sont cultivées dans des plaques à 96 puits, à une densité de 1, 2 ou 5 x 10⁵ cellules par ml en milieu RPMI-1640 ou en milieu MEM contenant 1%, 2%, 5 % ou 10% de sérum de veau foetal (SVF), 100 Unité / ml de pénicilline, 100 µg/ml de streptomycine, 200 mM de L-Glutamine à 37°C en incubateur à CO2. Le milieu de culture peut être remplacé tous les deux jours.

Dans le présent exemple, l'accumulation de vésicules lipidiques au sein de la cellule a été mesurée en utilisant des cellules THP1 après fixation par la paraformaldehyde en milieu PBS à l'aide d'une solution contenant un marqueur fluorescent de type Oil Red O pour visualiser les vésicules. L'image des cellules riches en vésicules a été analysée à l'aide d'un microscope équipé d'une caméra CCD et des logiciels nécessaires à l'analyse.

Les cellules THP-1 (5.10⁵ cellules/ml) (ECACC) étaient maintenues et cultivées dans du milieu RPMI-1640 contenant 10% de sérum de veaux foetal (FBS), 200 mM de L-Glutamine, 100 Unités/ml de pénicilline et 100 µg/ml de Streptomycine (Invitrogen-Life Technologies) à 37°C, dans un incubateur à 5% CO₂. Le milieu était remplacé tous les 2-3 jours.

Pour induire la différentiation des THP-1, 1.25 10⁵ cellules/puits étaient déposées dans des puits d'une plaque de culture 96 puits, dans leur milieu de culture contenant 10⁻⁷ M de phorbol 12-myristate-13-acetate (Sigma), pendant 24 heures à 37°C, 5% CO2. Les THP-1 différenciées étaient alors incubées avec des LDLox couplées à la cyanine-3 (1.5 µg/ml) en présence ou en absence de la molécule CGP02-01 (concentrations comprises entre 10⁻⁵M et 3.16 10⁻¹⁰M) pendant 24 heures à 37°C, 5% CO₂. Après fixation des cellules au paraformaldéhyde 4%, les noyaux étaient marqués au Hoechst 33342 (10 µg/ml) pendant 20 minutes, à température ambiante. Après deux lavages, 16 images/puits du signal liée à la cyanine-3 et au Hoechst 33342 étaient prises en utilisant un microscope à fluorescence couplé à une caméra CCD. Chaque image était analysée et quantifiée avec le logiciel MetaMorph (Universal Imaging).

Le tableau 1 ci-dessous rapporte le pourcentage observé pour l'inhibition de la captation et de l'accumulation sous forme de vésicules lipidiques, de lipoprotéines marquées à la cyanine 3, par des cellules exprimant le scavenger CD36. Les cellules ont été incubées en présence de chacune des molécules constituant cette famille et représentées par la molécule CGP02-01 à une concentration finale et identique pour chaque molécule de 2,5 µM.

**Tableau 1**

| Molécules | Inhibition de l'accumulation des LDL ox en ( % ) |
|---|---|
| CGP02-01 | 76 |
| CGP02-02 | 64 |
| CGP02-03 | 82 |
| CGP02-07 | 63 |
| CGP02-08 | 58 |
| CGP02-18 | 78 |

Le tableau de l'annexe 1 donne les structures de composés de l'invention ainsi que leur code par rapport au tableau 1 ci-dessus ainsi que leur pourcentage d'inhibition à une concentration de 25 microM sur les cellules THP1 pendant 24 heures.

Un des composés préféré de cette famille est le composé CGP 02-01. Lorsque des cellules macrophages de types THP1 différenciées sont cultivées en présence de ce composé à une concentration de 1 µM on observe une forte inhibition de l'accumulation de vésicules lipidiques (figure 1) Cet effet inhibiteur dépend de la concentration en produit CGP 02-01 (figure 1).

Bien que le composé CGP 02-01 ait été sélectionné comme produit de référence, d'autres composés de la même famille montrent la même activité et sont capables de bloquer l'accumulation de lipides intracellulaires. La figure 7 illustre l'activité inhibitrice des composés CGP 02-02 et CGP 02-03 faisant partis de la même famille de molécules.

### Exemple 3: Traitement de souris athéromateuse.

Différents types d'animaux peuvent être utilisés pour étudier les modifications du métabolisme des lipides, la formation de lésions artérielles et la progression d'une plaque d'athérome. Ces animaux sont disponibles dans le commerce. Il est possible d'utiliser des souris, des rats, des lapins hyperlipemiques (HWWL) ou des animaux plus importants comme le porc ou le singe. Des animaux génétiquement modifiés peuvent également être utilisés comme par exemple des souris ApoE -/- , LDL-R -/-, ApoAI -/-.

Deux types de modèles animaux ont été utilisés.

En premier lieu, des souris dépourvues du gène codant pour l'Apo lipoprotéine E (ApoE ^{-/-}) ont été utilisées. Ces souris représentent un modèle de choix pour l'étude de l'athérosclérose précoce et le développement d'une plaque riche en macrophages spumeux. Des souris males C57BL/6J homozygotes pour la délétion du gène ApoE ont été soumises à un régime normal jusqu' à l'age de huit semaines. Ces souris (n=8 par groupe) ont ensuite reçu ad libitum et pendant 12 semaines, soit un régime non enrichi en cholestérol ou en graisse, soit un régime enrichi, contenant 1,5 g/kg de cholestérol et 200 g/kg de graisse d'origine lactée. Les souris non traitées ont subi des injections quotidiennes intra péritonéale d'une solution contenant du DMS0 à 10%. Les souris traitées ont reçu par injection intra péritonéale, la même solution contenant 2 ou 20 µg (soit, 0,1 mg/kg/jour ou 1 mg/kg/jour) du composé CGP 02-01. Après le prélèvement de sang pour les analyses biochimiques, les souris ont été euthanasiées.

En second lieu, l'activité du composé CGP 02-01 a été examinée en utilisant un modèle de rat-fructose de référence. Des groupes de rats (n=12) Wistar, ont été soumis à un régime contenant 10% de fructose pendant 3 semaines. Dans ces conditions, les rats développent un syndrome métabolique comportant une hyperglycémie, une hyper insulinémie, une hypercholestérolémie et une hypertriglycéridémie. Des groupes de rats ont ensuite reçu par gavage 50 mg/kg du composé CGP 02-01 dissout dans une solution de tween 80 2% et formulé dans du methylcellulose. Les prélèvements de sang pour les analyses biochimiques ont été réalisés à 1, 2 et 3 semaines. Des groupes indépendants de rats ont été traités dans les mêmes conditions par du chlorhydrate de metformine à une dose de 50 mg/kg. La metformine a servi de référence dans ce modèle de syndrome métabolique.

### Exemple 4: Mesure du cholestérol libre dans le plasma.

La mesure du cholestérol libre peut se faire par une méthode enzymatique. Le cholestérol libre est oxydé par la cholestérol oxydase en Delta 4 cholestenone et produit de façon simultanée du péroxyde d'hydrogène. L'hydrogène peroxyde permet ensuite la condensation oxydative de la DHESA et de l'aminoantipyrine produisant une couleur bleue. La quantité de cholestérol libre est alors mesurée par l'absorbance de la couleur bleue. Les échantillons peuvent être récupérés sur un tampon citrate contenant de l'EDTA et de l'héparine. Cet essai peut être obtenu sous forme de kit dans le commerce.

Lorsque des souris ApoE ^{-/-} sont soumises à un régime normal non enrichi et traitées avec le composé CGP 02-01 (1 mg/kg), le taux plasmatique de cholestérol non estérifié diminue de manière significative. La variation observée chez les animaux non traités sur une période de 3 mois est en moyenne de 136 ± 19 g/L. La variation observée pour les animaux traités pendant la même période est de 105 ± 6 g/L, soit un effet de 22,7 % (p< 0,05).

### Exemple 5: Mesure du Cholestérol total dans le plasma.

Le cholestérol total circulant peut être mesuré par un dosage enzymatique à l'aide de kit existant dans le commerce. Ce dosage peut par exemple utiliser une séquence enzymatique du type cholestero-esterase/ cholesteroloxydase / péroxydase chromogène. En résumé, le cholestérol esterifié total est transformé en cholestérol libre et acide gras par l'action de la cholestérol esterase. Le cholestérol non estérifié est ensuite mesuré par la formation de quinoneimine en présence de cholestérol oxydase, et de péroxydase. L'intensité de la coloration qinoneimine est proportionnelle à la quantité de cholestérol présente dans l'échantillon.

Le tableau 2 ci-dessous rapporte les variations des taux plasmatiques du HDL et du cholesterol total chez une souris ApoE^{-/-} soumise à un régime riche en cholesterol et traitée par le composé CGP 02-01. Lorsque des souris Apode ^{-/-} sont traitées par CGP 02-01 (1 mg/kg) pendant 3 mois, le taux plasmatique de cholestérol total diminue de façon plus significative que chez les animaux non traités. Lorsque des souris sont soumises à un régime riche en cholestérol, le taux de cholestérol total passe d'une valeur de 7,27 ± 0,55 g/L à une valeur de 6,86 ± 0,65 g/L, soit une variation de 5,6%. Cet effet observé est dépendant de la dose de CGP 02-01.

**Tableau 2**

| | Taux plasmatique du HDL (g/L) | Taux plasmatique du cholesterol total (g/L) |
|---|---|---|
| Non traitées | 0,11 ± 0,05 | 7,27 ± 0,55 |
| 0,1 mg/Kg | 0,15 ± 0,06 | 8,08 ± 0,62 |
| 1 mg/Kg | 0,15 ± 0,05 | 6,86 ± 0,65 |

Dans le modèle de rat soumis à un régime riche en fructose, traité par voie orale (50 mg/kg), le composé CGP 02-01 produit une réduction très significative (p< 0,01) du taux de cholestérol total qui passe d'une valeur de 0,79 ± 0,05 g/L à une valeur de 0,36 ± 0,03 g/L soit une réduction de 54,5 % (p< 0,01) après 3 semaines de traitement. La metformine administrée par voie orale dans les mêmes conditions (50 mg/kg), produit une réduction du cholestérol total de 16% avec une valeur moyenne de 0,66 ± 0,02 g/L (Figure 12).

### Exemple 6: Mesure des triglycérides circulant.

Le dosage des triglycérides sériques peut être réalisé par voie enzymatique à l'aide de kit existant dans le commerce. On peut utiliser par exemple des kits de chez Biomerieux (ref. 61.238). En résumé, les triglycérides sont traités par une lipase pour générer du glycérol et des acides gras. En présence d'ATP, le glycérol est transformé en glycérol 3 phosphate par de la glycerokinase. Le glycérol 3 phosphate est ensuite transformé en dihydroxyacetone en générant de l'eau oxygénée (H2O2) qui peut être détectée par la formation de quinonéimine en présence de parachlorophénol, d'amino-4-antipyrine et de peroxydase. L'intensité de la coloration quinonéimine est alors mesurée à 505 nm. Cette coloration est proportionnelle à la quantité de triglycérides présents dans l'échantillon.

Lorsque des souris ApoE ^{-/-} sont soumises à un régime riche en cholestérol et en graisse et sont traitées pendant 3 mois par le composé CGP 02-01 (1mg/kg) leur taux de triglycérides plasmatiques varie et cette variation est 2 fois plus importante pour les souris traitées que pour les souris non traitées. Cette variation passe de -0,67 ± 0,54 g/L pour les souris non traitées à - 1,49 ± 0,57 g/L (p< 0,01) pour les souris traitées (figure 9).

Lorsque le composé CGP 02-01 est administré par voie orale à des rats sous régime riche en fructose, leur taux de triglycérides plasmatiques passe de 1,39 ± 0,13 g/L à 0,47 ± 0,07 g/L soit une variation de 66,2% (p<0,01). Dans les mêmes conditions, la metformine n'a pas d'effet, avec une valeur moyenne de 1,21 ± 0,08 g/L (figure 10).

### Exemple 7: Mesure de l'insuline dans le plasma

Le dosage de l'insuline dans le plasma peut être réalisé par dosage radio immunologique à l'aide de kits commerciaux comportant des anti corps spécifiques anti insuline de souris ou de rat. On peut utiliser par exemple les kits ELISA rat/souris , Linco research (ref EZRMI-13K).

Lorsque des souris ApoE ^{-/-} soumises à un régime riche en cholestérol son traitées par le composé CGP 02-01 à des doses de 0,1 ou 1 mg/kg on observe une réduction significative (p<0,01) du taux plasmatique d'insuline qui passe d'une valeur de 1,17 ± 0,2 ng/mL à une valeur de 0,95 ± 0,16, soit une variation de 18,8% (figure 11).

De manière identique, le composé CGP02-01 fait passer le taux d'insuline de rats soumis à un régime riche en fructose de la valeur 1,85 ± 0,04 à une valeur de 1,64 ± 0,03, soit une variation de 11,3% Dans les mêmes conditions, la metformine produit une réduction du taux d'insuline de 16,2%, (figure 12).

### Exemple 8: Mesure du taux de HDL dans le plasma

Le taux de HDL plasmatique est mesuré par des méthodes commerciales éprouvées qui utilisent des réactifs de séparation des lipoprotéines de haute densité et par une mesure du taux de cholestérol associé à ces lipoprotéines de haut poids moléculaire (kit de Biomérieux ref 61533 par exemple)

Lorsque des souris soumises à un régime riche en cholestérol et en acide gras sont traitées par le composé CGP 02-01 leur taux de HDL plasmatique passe de 0,11± 0, 005 g/L à 0,15± 0, 005 g/L soit une variation de 38,2%.

### Exemple 9: Mesure de la masse graisseuse abdominale

Des souris ApoE soumises à un régime riche en cholestérol et en graisse ont été sacrifiées après 3 mois de traitement par le composé CGP 02-01 aux doses de 0,1mg/kg et de 1 mg/kg. La masse graisseuse abdominale a été récupérée par dissection, séchée et exprimée en poids sec.

Le composé CGP 02-01 produit une réduction significative (p<0,01) de la masse de gras abdominal à gain de poids constant. Cette masse abdominale passe d'une valeur de 760 ± 231 mg à une valeur de 393 ± 78 mg lorsque les souris sont traitées à 1 mg/kg, soit une réduction de 48,3% (figure 13). Cet effet dépend de la dose de CGP 02-01.

### Exemple 10: Dépôt de triglycérides dans les aortes

Les triglycérides qui s'accumulent au niveau de la paroi aortique ont été mesurés de la manière suivante : les aortes des animaux sont rincées avec un sérum physiologique après dissection. La masse lipidique de l'adventice est éliminée par dissection, et l'intima media est déshydratée. Le taux de triglycérides est mesuré et exprimé en poids de triglycérides par poids sec de tissu.

Lorsque des souris ApoE ^{-/-} sont traitées par le composé CGP 02-01 pendant trois mois à une dose de 1mg/kg le dépôt de triglycérides sur la paroi aortique passe d'une valeur moyenne (n=8) de 185 ± 33 µg/mg (poids sec) à une valeur de 131 ± 42 µg/mg (poids sec). Cet effet dépend de la dose injectée. Une injection de 0,1 mg/kg produit une variation intermédiaire de 156 ± 31 µg/mg. La figure 14 illustre ce résultat.

### Exemple 11: Analyse des lésions aortiques

Les aortes des souris ont été fixées à la paraformaldehyde et disséquées en sections de 10µm pour l'analyse histologique des lésions (figure 6).

### Exemple 12: Analyse des ischémies coronariennes

Après euthanasie des souris ApoE -/- soumises à un régime normal ou un régime riche en cholestérol, les coeurs de ces animaux sont observés macroscopiquement. Le taux de lésions ischémiques est observé (figure 15) et quantifié par la présence ou l'absence de lésions (figure 16).

### Exemple 13: Mesure du taux plasmatique de glucose dans un modèle de rat diabétique

La glycémie est mesurée par la méthode à l'hexokinase à l'aide de kits commerciaux. On peut utiliser par exemple le kit Biomérieux (ref :61 269/61 270)

Lorsque des rats sont soumis à un régime riche en fructose, leur glycémie augmente en fonction du temps et passe d'une valeur moyenne (n=12) de 6,4 ± 0,15 mmole / L à une valeur moyenne 10,65 ± 0,24 mmole /L (figure 14). Le composé CGP 02-01 stabilise cette augmentation de la glycémie après 21 jours de traitement par voie orale (figure 17).

### Exemple 14: Mesure de l'effet protecteur lors d'une épreuve d'hyperglycémie

Des rats (n=12) ont été soumis à un régime riche en fructose (10%) pendant 21 jours. Au bout du 14éme jour, un choc hyperglycémique est provoqué par une administration de 2 g/kg de glucose en présence ou en absence du composé CGP 02-01 administré par voie orale. Le taux plasmatique de glucose est ensuite mesuré. La figure 18 illustre l'effet protecteur du produit sur l'hyperglycémie provoquée.

| **Structure** | **Composés** | **Inhibition à 2.5 µM sur les cellules THP-1 (24 heures)** |
|---|---|---|
| 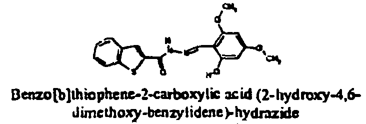 | CGP02-01 | 76% |
| 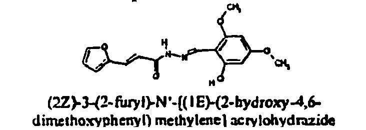 | CGP02-02 | 64% |
| 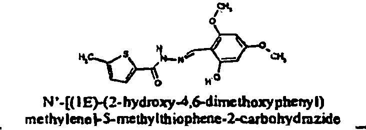 | CGP02-03 | 82% |
| 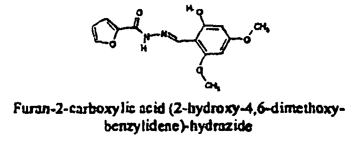 | CGP02-07 | 63% |
| 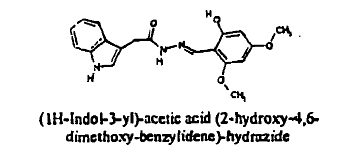 | CGP02-08 | 58% |
| 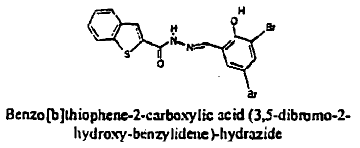 | CGP02-18 | 78% |

## Revendications

1. Un composé de formule générale (IV) suivante : 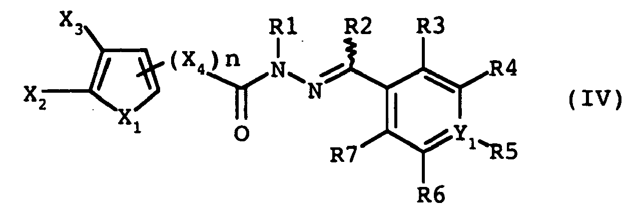 dans laquelle :
- R1 et R2, identiques ou différents, sont choisis parmi un atome d'hydrogène, un radical alkyle inférieur linéaire ou ramifié de 1 à 6 atomes de carbone, un radical fluoroalkyle de 1 à 6 atomes de carbones et de 3 à 7 atomes de fluor,
- R3, R4, R5, R6 et R7, identiques ou différents, sont choisis parmi : un atome d'hydrogène, un atome d'halogène, un groupe de formule -OH, -OR8 ou -OCOR9, où R8 et R9 représentent un radical alkyle inférieur linéaire ou ramifié de 1 à 6 carbones, un groupe amino -NH₂ ou -N(r, r') dans lequel r et r' identiques ou différents représentent un radical alkyle inférieur linéaire ou ramifié, un radical aryle, ou un hétérocycle dans lequel r et r' pris ensemble forment un hétérocycle de taille variable, de préférence en position *para,*
- Y₁ est un atome de carbone pour former un noyau phényle ou un atome d'azote pour former un noyau pyridine,
- X₁ est choisi parmi :
• un atome d'oxygène et dans ce cas le groupement de formule (III) suivant : 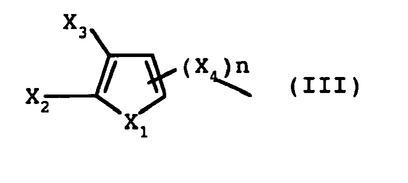 est un noyau 2-furanyl ou 3-furanyl en fonction de la position de la chaîne -(X₄)ₙ-acyl-hydrazide sur les carbones α ou β de cet hétérocycle,
• un atome de soufre et dans ce cas le groupement de formule (III) est un noyau 2-thiophène ou 3-thiophène en fonction de la position de la chaîne -(X₄)ₙ-acyl-hydrazide sur les carbones α ou β, cet atome de soufre pouvant porter un atome d'oxygène pour former un sulfoxyde ou deux atomes d'oxygène pour former une sulfone,
• un atome d'azote et dans ce cas le groupement de formule (III) est un noyau 2-pyrrol ou 3-pyrrol en fonction de la position de la chaîne acyl-hydrazide sur les carbones α ou β de cet hétérocycle, cet atome d'azote pouvant porter un atome d'hydrogène, un radical alkyle inférieur de 1 à 6 atomes de carbones, un radical fluoroalykyle ayant de 1 à 6 atomes de carbone et de 3 à 7 atomes de fluor, un radical acyle -COR10 dans lequel R10 représente une chaîne alkyle linéaire ou ramifiée de 1 à 6 carbones, ou un radical aryle ou aralkyle,
- X₂ et X₃, identiques ou différents, sont choisis parmi:
• un atome d'hydrogène, un groupe alkyle linéaire ou ramifiée inférieure de 1 à 6 atomes de carbone, un radical fluoroalkyle ayant de 1 à 6 atomes de carbone et de 3 à 7 atomes de fluor,
• un atome d'halogène,
• un groupe nitro -NO₂, un groupe amino -NH₂ ou un groupe -N(r, r'), dans lequel r et r' identiques ou différents représentent un radical alkyle inférieur linéaire ou ramifié, un radical aryle, ou un hétérocycle dans lequel r et r' pris ensemble forment un hétérocycle de taille variable,
ou encore X₂ et X₃ sont inclus dans un cycle aromatique de type benzénique ou aza-benzénique si ce cycle comporte un atome d'azote, pour former un hétérocycle aromatique de type benzofurane lorsque X₁ est un atome d'oxygène, un noyau benzopyrrole lorsque X₁ est un atome d'azote libre ou substitué comme ci-dessus, un noyau benzothiophène lorsque X₁ est un atome de soufre libre ou substitué comme ci-dessus ou encore un noyau de type pyridino si un atome d'azote intracyclique est présent,
- n est 0 ou 1,
- X₄, s'il est présent, représente un groupe -CH₂-, -OCH₂-, ou -CH=CH-.

2. Un composé de formule (IV) selon la revendication 1, dans laquelle R3 est un groupe de formule -OR8 et au moins deux des substituants R4, R5, R6 et R7 représentent un atome d'hydrogène.

3. Un composé de formule (IV) selon l'une des revendications 1 ou 2, dans laquelle Y₁ est un atome de carbone.

4. Un composé de formule (IV) selon la revendication 1, dans laquelle X₁ est un atome de soufre, et dans ce cas le groupement de formule (III) 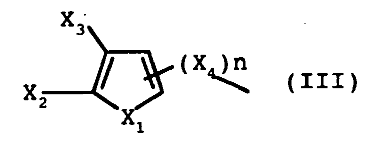 est un noyau 2-thiophène ou 3-thiophène en fonction de la position de la chaîne -(X₄)ₙ-acyl-hydrazide sur les carbones α
ou β, cet atome de soufre pouvant porter un atome d'oxygène pour former un sulfoxyde ou deux atomes d'oxygène pour former une sulfone.

5. Un composé selon la revendication 1, choisi dans le groupe comprenant :
• N'-[(*1E*)-(2-hydroxy-4,6 diméthoxyphényl)méthylène]-1-benzothiophène-2-carbohydrazide,
• (*2Z*)-3-(2-furyl)-N'-[(*1E*)-(2-hydroxy-4,6-diméthoxyphényl)méthylène] acrylohydrazide,
• N'-[(1E)-(2-hydroxy-4,6-diméthoxyphényl)méthylène)-5-méthylthiophène-2-carbohydrazide,
• 2-furancarboxylique acide (2-hydroxy-4,6-diméthoxy-benzylidène)-hydrazide,
• (1H-indol-3-yl) acétique acide(2-hydroxy-4,6-diméthoxybenzylidène)-hydrazide,
• benzo[b]thiophène-2-carboxylique acide (3,5-dibromo-2-hydroxy-benzylidene)-hydrazide.

6. Le N'-[(*1E*)-(2-hydroxy-4,6-diméthoxyphényl) méthylène]-1-benzothiophène-2-carbohydrazide.

7. Un composé de formule (IV) selon l'une quelconque des revendications 1 à 3, dans lequel le groupe de formule (III) : 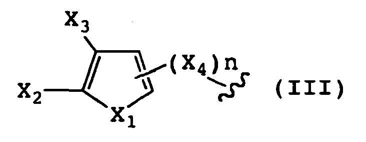 représente un groupement de formule (IX) suivante : 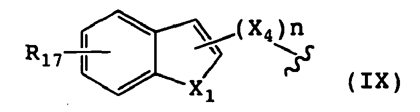 dans laquelle :
- X₁ et X₄ ont la même signification que dans les revendications précédentes,
- n est 0 ou 1,
- R17 est choisi parmi :
• un atome d'hydrogène, un radical alkyle inférieur linéaire ou ramifié de 1 à 6 atomes de carbone, un radical fluoroalkyle de 1 à 6 atomes de carbones et de 3 à 7 atomes de fluor,
• un atome d'halogène,
• un groupement OR' pour lequel R' inférieur linéaire ou ramifié de 1 à 6 atomes de carbone, un radical fluoroalkyle de 1 à 6 atomes de carbone et de 3 à 7 atomes de fluor.

8. Un sel d'un composé selon l'une quelconque des revendications précédentes avec un acide pharmaceutiquement acceptable.

9. Composition pharmaceutique comprenant à titre d'agent actif au moins un composé selon l'une quelconque des revendications 1 à 8.

10. Composition selon la revendication 9, laquelle est destinée au traitement et/ou à la prévention des maladies associées à des désordres du métabolisme des lipides.

11. Composition selon l'une des revendications 9 ou 10, laquelle est destinée au traitement et/ou à la prévention des maladies cardiovasculaires.

12. Composition selon l'une quelconque des revendications 9 à 11, laquelle est destinée au traitement et/ou à la prévention d'une maladie choisie dans le groupe comprenant l'athérosclérose, la resténose artérielle, l'obésité, le diabète de type II, l'ischémie cérébrale, les stéatoses hépatiques, l'hypercholestérolémie, l'hypertriglycéridémie , la dyslipoproteinémie, la chylomicronémie, la lipodistrophie, l'hyperglycémie.

13. Utilisation d'un composé selon l'une quelconque des revendications 1 à 8 pour la fabrication d'un médicament.

## Claims

1. Compound of general formula (IV) below: 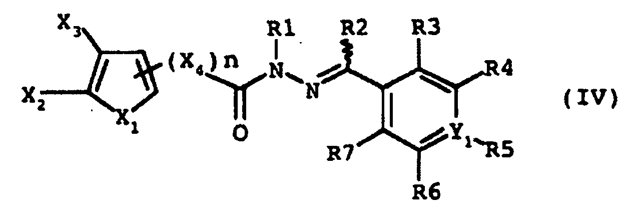 in which:
- R1 and R2, which may be identical or different, are chosen from a hydrogen atom, a linear or branched lower alkyl radical of 1 to 6 carbon atoms and a fluoroalkyl radical of 1 to 6 carbon atoms and of 3 to 7 fluorine atoms,
- R3, R4, R5, R6 and R7, which may be identical or different, are chosen from: a hydrogen atom, a halogen atom, a group of formula -OH, -OR8 or -OCOOR9, in which R8 and R9 represent a linear or branched lower alkyl radical of 1 to 6 carbons, an amino group -NH₂ or -N(r, r') in which r and r', which may be identical or different, represent a linear or branched lower alkyl radical, an aryl radical, or a heterocycle in which r and r', taken together, form a heterocycle of variable size, preferably in the para position,
- Y₁ is a carbon atom to form a phenyl nucleus or a nitrogen atom to form a pyridine nucleus,
- X₁ is chosen from:
• an oxygen atom, and in this case the group of formula (III) below: 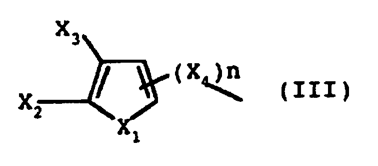 is a 2-furyl or 3-furyl nucleus depending on the position of the chain - -(X₄)ₙ-acyl-hydrazide on the α or β carbons of this heterocycle,
• a sulfur atom, and in this case the group of formula (III) is a 2-thiophene or 3-thiophene nucleus depending on the position of the chain -(X₄)ₙ-acyl-hydrazide on the α or β carbons, this sulfur atom possibly bearing an oxygen atom to form a sulfoxide or two oxygen atoms to form a sulfone,
• a nitrogen atom, and in this case the group of formula (III) is a 2-pyrrole or 3-pyrrole nucleus depending on the acyl-hydrazide chain on the α or β carbons of this heterocycle, this nitrogen atom possibly bearing a hydrogen atom, a lower alkyl radical of 1 to 6 carbon atoms, a fluoroalkyl radical containing from 1 to 6 carbon atoms and from 3 to 7 fluorine atoms, a radical acyl-COR10 in which R10 represents a linear or branched alkyl chain of 1 to 6 carbons, or an aryl or aralkyl radical,
- X₂ and X₃, which may be identical or different, are chosen from:
• a hydrogen atom, a linear or branched lower alkyl group of 1 to 6 carbon atoms or a a fluoroalkyl radical containing from 1 to 6 carbon atoms and from 3 to 7 fluorine atoms,
• a halogen atom,
• a nitro group -NO₂, an amino group -NH₂ or a group -N(r, r'), in which r and r', which may be identical or different, represent a linear or branched lower alkyl radical, an aryl radical, or a heterocycle in which r and r', taken together, form a heterocycle of variable size,
or alternatively X₂ and X₃ are included in an aromatic ring of benzene or azabenzene type if this ring bears a nitrogen atom, to form an aromatic heterocycle of benzofuran type when X₁ is an oxygen atom, a benzopyrrole nucleus when X₁ is a free or substituted nitrogen atom as above, a benzothiophene nucleus when X₁ is a free or substituted sulfur atom as above, or a nucleus of pyridino type if an endocyclic nitrogen atom is present,
- n is 0 or 1,
- X₄, if it is present, represents a group -CH₂-,
- OCH₂- or -CH=CH-.

2. Compound of formula (IV) according to Claim 1, in which R3 is a group of formula -OR8 and at least two of the substituents R4, R5, R6 and R7 represent a hydrogen atom.

3. Compound of formula (IV) according to either of Claims 1 and 2, in which Y₁ is a carbon atom.

4. Compound of formula (IV) according to Claim 1, in which X₁ is a sulfur atom, and, in this case, the group of formula (III) 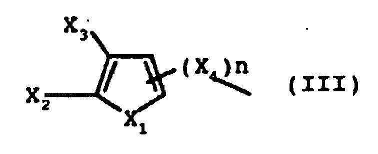 is a 2-thiophene or 3-thiophene nucleus depending on the position of the chain -(X₄)ₙ-acyl-hydrazide on the α or β carbons of this heterocycle, this sulfur atom possibly bearing an oxygen atom to form a sulfoxide, or two oxygen atoms to form a sulfone.

5. Compound according to Claim 1, chosen from the group comprising:
• N'-[(1*E*)-(2-hydroxy-4,6-dimethoxyphenyl)methylene]-1-benzothiophene-2-carbohydrazide,
• (2*Z*)-3-(2-furyl)-N'-[(1*E*)-(2-hydroxy-4,6-dimethoxyenyl)methylene]acrylohydrazide,'
• N'-[(1*E*)-(2-hydroxy-4,6-dimethoxyphenyl)methylene]-5-methylthiophene-2-carbohydrazide,
• 2-furancarboxylic acid (2-hydroxy-4,6-dimethoxy benzylidene)hydrazide,
• (1H-indol-3-yl)acetic acid (2-hydroxy-4,6-dimethoxy benzylidene)hydrazide,
• benzo[b]thiophene-2-carboxylic acid (3,5-dibromo-2-hydroxybenzylidene)hydrazide.

6. N'-[(1*E*)-(2-Hydroxy-4,6-dimethoxyphenyl)methylene]-1-benzothiophene-2-carbohydrazide.

7. Compound of formula (IV) according to any one of Claims 1 to 3, in which the group of formula (III): 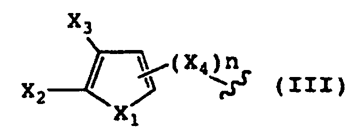 represents a group of formula (IX) below: 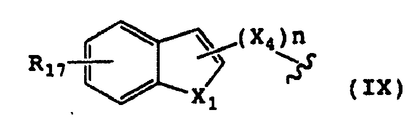 in which:
- X₁ and X₄ have the same meaning as in the preceding claims,
- n is 0 or 1,
- R₁₇ is chosen from:
• a hydrogen atom, a linear or branched lower alkyl radical of 1 to 6 carbon atoms or a fluoroalkyl radical of 1 to 6 carbon atoms and of 3 to 7 fluorine atoms,
• a halogen atom,
• a group OR' for which R' is a linear or branched lower alkyl radical of 1 to 6 carbon atoms or a fluoroalkyl radical of 1 to 6 carbon atoms and of 3 to 7 fluorine atoms.

8. Salt of a compound according to any one of the preceding claims with a pharmaceutically acceptable acid.

9. Pharmaceutical composition comprising, as active agent, at least one compound according to any one of Claims 1 to 8.

10. Composition according to Claim 9, which is intended for treating and/or preventing diseases associated with fat metabolism disorders.

11. Composition according to either of Claims 9 and 10, which is intended for treating and/or preventing cardiovascular diseases.

12. Composition according to any one of Claims 9 to 11, which is intended for treating and/or preventing diseases chosen from the group comprising atherosclerosis, arterial restenosis, obesity, type II diabetes, cerebral ischaemia, hepatic steatosis, hypercholesterolaemia, hypertriglyceridaemia, dyslipoproteinaemia, chylomicronaemia, lipodystrophy and hypoglycaemia.

13. Use of a compound according to any one of Claims 1 to 8, for the manufacture of a medicament.

## Patentansprüche

1. Verbindung der folgenden allgemeinen Formel (IV): 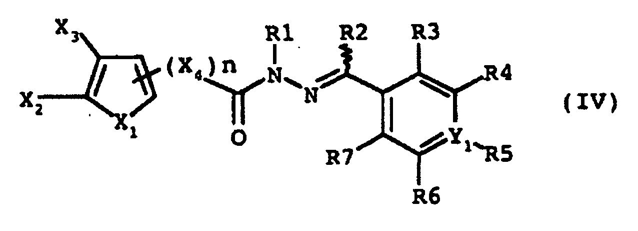 worin:
- R1 und R2 gleich oder verschieden sind und unter einem Wasserstoffatom, einem linearen oder verzweigten Niederalkylrest mit 1 bis 6 Kohlenstoffatomen und einem Fluoralkylrest mit 1 bis 6 Kohlenstoffatomen und 3 bis 7 Fluoratomen ausgewählt sind,
- R3, R4, R5, R6 und R7 gleich oder verschieden sind und ausgewählt sind unter: einem Wasserstoffatom, einem Halogenatom, einer Gruppe der Formel
- OH, -OR8 oder -OCOR9, wobei R8 und R9 für einen linearen oder verzweigten Niederalkylrest mit 1 bis 6 Kohlenstoffatomen stehen, und einer Aminogruppe -NH₂ oder -N(r, r'), worin r und r' gleich oder verschieden sind und für einen linearen oder verzweigten Niederalkylrest, einen Arylrest oder einen Heterocyclus, in dem r und r' zusammengenommen einen Heterocyclus variabler Größe bilden, vorzugsweise in para-Position, stehen,
- Y₁ für ein Kohlenstoffatom zur Bildung eines Phenylkerns oder ein Stickstoffatom zur Bildung eines Pyridinkerns steht,
- X₁ ausgewählt ist unter:
• einem Sauerstoffatom, wobei die Gruppierung der folgenden Formel (III): 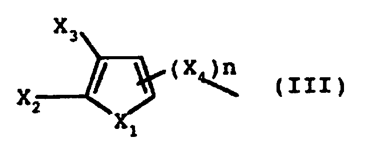 je nach der Position der -(X₄)ₙ-Acylhydrazid-Kette am α- oder β-Kohlenstoffatom dieses Heterocyclus für einen 2-Furanyl- oder 3-Furanylkern steht,
• einem Schwefelatom, wobei die Gruppierung der Formel (III) je nach der Position der -(X₄)ₙ-Acylhydrazid-Kette am α- oder β-Kohlenstoffatom für einen 2-Thiophen- oder 3-Thiophenkern steht, wobei dieses Schwefelatom ein Sauerstoffatom zur Bildung eines Sulfoxids oder zwei Sauerstoffatome zur Bildung eines Sulfons tragen kann,
• einem Stickstoffatom, wobei die Gruppierung der Formel (III) je nach der Position der Acylhydrazid-Kette am α- oder β-Kohlenstoffatom dieses Heterocyclus für einen 2-Pyrrol- oder 3-Pyrrolkern steht, wobei dieses Stickstoffatom ein Wasserstoffatom, einen Niederalkylrest mit 1 bis 6 Kohlenstoffatomen, einen Fluoralkylrest mit 1 bis 6 Kohlenstoffatomen und 3 bis 7 Fluoratomen, einen Acylrest -COR10, worin R10 für eine lineare oder verzweigte Alkylkette mit 1 bis 6 Kohlenstoffatomen steht, oder einen Aryl- oder Aralkylrest tragen kann,
- X₂ und X₃ gleich oder verschieden sind und ausgewählt sind unter:
• einem Wasserstoffatom, einer linearen oder verzweigten Niederalkylgruppe mit 1 bis 6 Kohlenstoffatomen, einem Fluoralkylrest mit 1 bis 6 Kohlenstoffatomen und 3 bis 7 Fluoratomen,
• einem Halogenatom,
• einer Nitrogruppe -NO₂, einer Aminogruppe -NH₂ oder einer Gruppe -N(r, r'), worin r und r' gleich oder verschieden sind und für einen linearen oder verzweigten Niederalkylrest, einen Arylrest oder einen Heterocyclus, in dem r und r' zusammengenommen einen Heterocyclus variabler Größe bilden, stehen,
oder X₂ und X₃ auch Teil eines aromatischen Rings vom Benzol- oder Azabenzol-Typ, wenn dieser Ring ein Stickstoffatom enthält, zur Bildung eines aromatischen Heterocyclus vom Benzofuran-Typ, wenn X₁ für ein Sauerstoffatom steht, eines Benzopyrrolkerns, wenn X₁ für ein freies oder wie oben substituiertes Stickstoffatom steht, eines Benzothiophenkerns, wenn X₁ für ein freies oder wie oben substituiertes Schwefelatom steht, oder auch eines Kerns vom Pyridino-Typ, wenn ein intracyclisches Stickstoffatom vorliegt, sind,
- n für 0 oder 1 steht,
- X₄, sofern vorhanden, für eine -CH₂-, -OCH₂- oder
- CH=CH-Gruppe steht.

2. Verbindung der Formel (IV) nach Anspruch 1, worin R3 für eine Gruppe der Formel -OR8 steht und mindestens zwei der Substituenten R4, R5, R6 und R7 für ein Wasserstoffatom stehen.

3. Verbindung der Formel (IV) nach einem der Ansprüche 1 oder 2, worin Y₁ für ein Kohlenstoffatom steht.

4. Verbindung der Formel (IV) nach Anspruch 1, worin X₁ für ein Schwefelatom steht, wobei die Gruppierung der Formel (III) 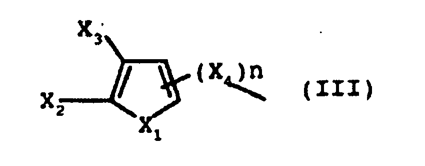 je nach der Position der -(X₄)ₙ-Acylhydrazid-Kette am α- oder β-Kohlenstoffatom für einen 2-Thiophen- oder 3-Thiophenkern steht, wobei dieses Schwefelatom ein Sauerstoffatom zur Bildung eines Sulfoxids oder zwei Sauerstoffatome zur Bildung eines Sulfons tragen kann.

5. Verbindung nach Anspruch 1, ausgewählt aus der Gruppe enthaltend:
• N'-(*1E*)-(2-Hydroxy-4,6-dimethoxyphenyl)-methylen]-1-benzothiophen-2-carbohydrazid,
• (*2Z*)-3-(2-Furyl-N'-[(*1E*)-(2-hydroxy-4,6-dimethoxyphenyl)methylen]acrylsäurehydrazid,
• N'-(*1E*)-(2-Hydroxy-4,6-dimethoxyphenyl)-methylen]-5-methylthiophen-2-carbohydrazid,
• 2-Furancarbonsäure(2-hydroxy-4,6-dimethoxy-benzyliden)hydrazid,
• (1H-Indol-3-yl)essigsäure(2-hydroxy-4,6-dimethoxybenzyliden)hydrazid,
• Benzo[b]thiophen-2-carbonsäure(3,5-dibrom-2-hydroxybenzyliden)hydrazid.

6. N'-(*1E*)-(2-Hydroxy-4,6-dimethoxyphenyl)methylen]-1-benzothiophen-2-carbohydrazid.

7. Verbindung der Formel (IV) nach einem der Ansprüche 1 bis 3, worin die Gruppe der Formel (III) : 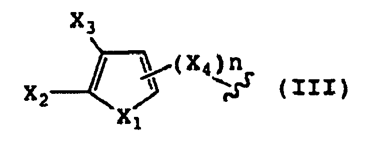 für eine Gruppe der folgenden Formel (IX) steht: 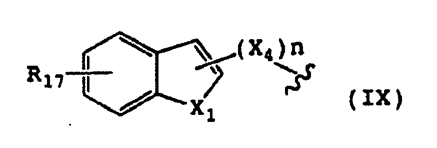 worin:
- X₁ und X₂ die gleiche Bedeutung wie in den vorhergehenden Ansprüchen besitzen,
- n für 0 oder 1 steht,
- R17 ausgewählt ist unter:
• einem Wasserstoffatom, einer linearen oder verzweigten Niederalkylgruppe mit 1 bis 6 Kohlenstoffatomen, einem Fluoralkylrest mit 1 bis 6 Kohlenstoffatomen und 3 bis 7 Fluoratomen,
• einem Halogenatom,
• einer Gruppe OR', wobei R' für eine lineare oder verzweigte Niederalkylgruppe mit 1 bis 6 Kohlenstoffatomen oder einen Fluoralkylrest mit 1 bis 6 Kohlenstoffatomen und 3 bis 7 Fluoratomen steht.

8. Salz einer Verbindung nach einem der vorhergehenden Ansprüche mit einer pharmazeutisch unbedenklichen Säure.

9. Pharmazeutische Zusammensetzung, die als Wirkstoff mindestens eine Verbindung nach einem der Ansprüche 1 bis 8 enthält.

10. Zusammensetzung nach Anspruch 9 zur Behandlung und/oder Prävention von Erkrankungen, die mit Störungen des Lipidstoffwechsels assoziiert sind.

11. Zusammensetzung nach einem der Ansprüche 9 oder 10 zur Behandlung und/oder Prävention von Herz-Kreislauf-Erkrankungen.

12. Zusammensetzung nach einem der Ansprüche 9 bis 11 zur Behandlung und/oder Prävention einer Erkrankung aus der Gruppe enthaltend Atherosklerose, arterielle Restenose, Obesitas, Typ-II-Diabetes, Hirnischämie, Lebersteatose, Hypercholesterinämie, Hypertriglyceridämie, Dyslipoproteinämie, Chylomikronämie, Lipodistrophie und Hyperglykämie.

13. Verwendung einer Verbindung nach einem der Ansprüche 1 bis 8 zur Herstellung eines Arzneimittels.
